# EUROPEAN PATENT APPLICATION

(11) **EP 2 605 016 A1**
(43) Date of publication of application: **19.06.2013**
(21) Application number: 11193519.3
(22) Date of filing: 14.12.2011
(51) Int. Cl.: G01N 33/574

(54) **Biomarkers related to lung cancer**

(71) Applicant: Philip Morris Products S.A., 2000 Neuchâtel (CH)
(72) Inventor: Bonk, Thomas, 50679 Köln (DE); Schlage, Walter K., 51429 Bergisch Gladbach (DE)
(74) Representative: Vossius & Partner

(57) **Abstract**

The present invention relates to a method for the detection of proteins and peptides associated with cancerous cells in lung tissue samples. More specifically, the invention relates to a method of detecting significant changes in the amount and/or chemical structure of peptides and/or proteins associated with lung tumor cells. The present invention further provides a method for early diagnosis and prognosis of lung cancer and for assessing the risk of getting lung cancer.

SEQUENCE LISTING

<110>  Philip Morris Products S.A.

<120>  Biomarkers related to lung cancer

<130>  N1275 EP BS

<160>  42

<170>  PatentIn version 3.4

<210>  1
<211>  31
<212>  PRT
<213>  Homo sapiens

<400>  1

Leu Trp Ile Leu Gly Asp Val Phe Ile Gly Arg Tyr Tyr Thr Val Phe
1               5                   10                  15

Asp Arg Asp Asn Asn Arg Val Gly Phe Ala Glu Ala Ala Arg Leu
            20                  25                  30

<210>  2
<211>  26
<212>  PRT
<213>  Homo sapiens

<400>  2

Asp Val Phe Ile Gly Arg Tyr Tyr Thr Val Phe Asp Arg Asp Asn Asn
1               5                   10                  15

Arg Val Gly Phe Ala Glu Ala Ala Arg Leu
            20                  25

<210>  3
<211>  41
<212>  PRT
<213>  Homo sapiens

<400>  3

Met Ser Asp Ala Ala Val Asp Thr Ser Ser Glu Ile Thr Thr Lys Asp
1               5                   10                  15

Leu Lys Glu Lys Lys Glu Val Val Glu Glu Ala Glu Asn Gly Arg Asp
            20                  25                  30

Ala Pro Ala Asp Glu Glu Asn Gly Glu
        35                  40

<210>  4
<211>  35
<212>  PRT
<213>  Homo sapiens

<400>  4

Ser Asp Ala Ala Val Asp Thr Ser Ser Glu Ile Thr Thr Lys Asp Leu
1               5                   10                  15

Lys Glu Lys Lys Glu Val Val Glu Glu Ala Glu Asn Gly Arg Asp Ala
            20                  25                  30

Pro Ala Asp
        35

<210>  5
<211>  85
<212>  PRT
<213>  Homo sapiens

<400>  5

Ala Glu Asp Met Glu Thr Lys Ile Lys Asn Tyr Lys Thr Ala Pro Phe
1               5                   10                  15

Asp Ser Arg Phe Pro Asn Gln Asn Gln Thr Arg Asn Cys Trp Gln Asn
            20                  25                  30

Tyr Leu Asp Phe His Arg Cys Gln Lys Ala Met Thr Ala Lys Gly Gly
        35                  40                  45

Asp Ile Ser Val Cys Glu Trp Tyr Gln Arg Val Tyr Gln Ser Leu Cys
    50                  55                  60

Pro Thr Ser Trp Val Thr Asp Trp Asp Glu Gln Arg Ala Glu Gly Thr
65                  70                  75                  80

Phe Pro Gly Lys Ile
                85

<210>  6
<211>  23
<212>  PRT
<213>  Homo sapiens

<400>  6

Gly Lys Val Lys Val Gly Val Asn Gly Phe Gly Arg Ile Gly Arg Leu
1               5                   10                  15

Val Thr Arg Ala Ala Phe Asn
            20

<210>  7
<211>  18
<212>  PRT
<213>  Homo sapiens

<400>  7

Gly Lys Val Lys Val Gly Val Asn Gly Phe Gly Arg Ile Gly Arg Leu
1               5                   10                  15

Val Thr

<210>  8
<211>  85
<212>  PRT
<213>  Homo sapiens

<400>  8

Ala Glu Asp Met Glu Thr Lys Ile Lys Asn Tyr Lys Thr Ala Pro Phe
1               5                   10                  15

Asp Ser Arg Phe Pro Asn Gln Asn Gln Thr Arg Asn Cys Trp Gln Asn
            20                  25                  30

Tyr Leu Asp Phe His Arg Cys Gln Lys Ala Met Thr Ala Lys Gly Gly
        35                  40                  45

Asp Ile Ser Val Cys Glu Trp Tyr Gln Arg Val Tyr Gln Ser Leu Cys
    50                  55                  60

Pro Thr Ser Trp Val Thr Asp Trp Asp Glu Gln Arg Ala Glu Gly Thr
65                  70                  75                  80

Phe Pro Gly Lys Ile
                85

<210>  9
<211>  23
<212>  PRT
<213>  Homo sapiens

<400>  9

Ala Tyr Leu Leu Gly Lys Glu Asp Ala Ala Arg Glu Ile Arg Arg Phe
1               5                   10                  15

Ser Phe Cys Cys Ser Pro Glu
            20

<210>  10
<211>  13
<212>  PRT
<213>  Homo sapiens

<400>  10

Lys Glu Asp Ala Ala Arg Glu Ile Arg Arg Phe Ser Phe

1               5                   10


<210>  11
<211>  30
<212>  PRT
<213>  Homo sapiens

<400>  11

His Phe Val Lys Leu Ile Ser Trp Tyr Asp Asn Glu Phe Gly Tyr Ser
1               5                   10                  15


Asn Arg Val Val Asp Leu Met Ala His Met Ala Ser Lys Glu
            20                  25                  30


<210>  12
<211>  25
<212>  PRT
<213>  Homo sapiens

<400>  12

Ile Ser Trp Tyr Asp Asn Glu Phe Gly Tyr Ser Asn Arg Val Val Asp
1               5                   10                  15


Leu Met Ala His Met Ala Ser Lys Glu
            20                  25


<210>  13
<211>  38
<212>  PRT
<213>  Homo sapiens

<400>  13

Val Asn Gly Phe Gly Arg Ile Gly Arg Leu Val Thr Arg Ala Ala Phe
1               5                   10                  15


Asn Ser Gly Lys Val Asp Ile Val Ala Ile Asn Asp Pro Phe Ile Asp
            20                  25                  30


Leu Asn Tyr Met Val Tyr
        35


<210>  14
<211>  28
<212>  PRT
<213>  Homo sapiens

<400>  14

Arg Ile Gly Arg Leu Val Thr Arg Ala Ala Phe Asn Ser Gly Lys Val
1               5                   10                  15


Asp Ile Val Ala Ile Asn Asp Pro Phe Ile Asp Leu
            20                  25

<210>  15
<211>  26
<212>  PRT
<213>  Homo sapiens

<400>  15

Val Pro Val Ser Glu Pro Val Pro Glu Pro Glu Pro Glu Pro Glu Pro
1               5                   10                  15

Glu Pro Val Lys Glu Glu Lys Leu Ser Pro
            20                  25

<210>  16
<211>  14
<212>  PRT
<213>  Homo sapiens

<400>  16

Pro Val Pro Glu Pro Glu Pro Glu Pro Glu Pro Glu Pro Val
1               5                   10

<210>  17
<211>  27
<212>  PRT
<213>  Homo sapiens

<400>  17

His Phe Val Lys Leu Ile Ser Trp Tyr Asp Asn Glu Phe Gly Tyr Ser
1               5                   10                  15

Asn Arg Val Val Asp Leu Met Ala His Met Ala
            20                  25

<210>  18
<211>  17
<212>  PRT
<213>  Homo sapiens

<400>  18

Ile Ser Trp Tyr Asp Asn Glu Phe Gly Tyr Ser Asn Arg Val Val Asp
1               5                   10                  15

Leu

<210>  19
<211>  101
<212>  PRT
<213>  Homo sapiens

<400>  19

Ser Glu Lys Ser Val Glu Ala Ala Ala Glu Leu Ser Ala Lys Asp Leu
1               5                   10                  15

Lys Glu Lys Lys Glu Lys Val Glu Glu Lys Ala Ser Arg Lys Glu Arg
            20                  25                  30

Lys Lys Glu Val Val Glu Glu Glu Glu Asn Gly Ala Glu Glu Glu Glu
        35                  40                  45

Glu Glu Thr Ala Glu Asp Gly Glu Glu Glu Asp Glu Gly Glu Glu Glu
    50                  55                  60

Asp Glu Glu Glu Glu Glu Glu Asp Asp Glu Gly Pro Ala Leu Lys Arg
65                  70                  75                  80

Ala Ala Glu Glu Glu Asp Glu Ala Asp Pro Lys Arg Gln Lys Thr Glu
                85                  90                  95

Asn Gly Ala Ser Ala
            100

<210>  20
<211>  97
<212>  PRT
<213>  Homo sapiens

<400>  20

Ser Glu Lys Ser Val Glu Ala Ala Ala Glu Leu Ser Ala Lys Asp Leu
1               5                   10                  15

Lys Glu Lys Lys Glu Lys Val Glu Glu Lys Ala Ser Arg Lys Glu Arg
            20                  25                  30

Lys Lys Glu Val Val Glu Glu Glu Glu Asn Gly Ala Glu Glu Glu Glu
        35                  40                  45

Glu Glu Thr Ala Glu Asp Gly Glu Glu Glu Asp Glu Gly Glu Glu Glu
    50                  55                  60

Asp Glu Glu Glu Glu Glu Glu Asp Asp Glu Gly Pro Ala Leu Lys Arg
65                  70                  75                  80

Ala Ala Glu Glu Glu Asp Glu Ala Asp Pro Lys Arg Gln Lys Thr Glu
                85                  90                  95

Asn

<210>  21
<211>  27

<212>  PRT
<213>  Homo sapiens

<400>  21

Met Ala Asn Leu Glu Arg Thr Phe Ile Ala Ile Lys Pro Asp Gly Val
1               5                   10                  15

Gln Arg Gly Leu Val Gly Glu Ile Ile Lys Arg
            20                  25

<210>  22
<211>  21
<212>  PRT
<213>  Homo sapiens

<400>  22

Ala Asn Leu Glu Arg Thr Phe Ile Ala Ile Lys Pro Asp Gly Val Gln
1               5                   10                  15

Arg Gly Leu Val Gly
            20

<210>  23
<211>  31
<212>  PRT
<213>  Homo sapiens

<400>  23

Met Ala Asp Ile Gln Thr Glu Arg Ala Tyr Gln Lys Gln Pro Thr Ile
1               5                   10                  15

Phe Gln Asn Lys Lys Arg Val Leu Leu Gly Glu Thr Gly Lys Glu
            20                  25                  30

<210>  24
<211>  25
<212>  PRT
<213>  Homo sapiens

<400>  24

Ala Asp Ile Gln Thr Glu Arg Ala Tyr Gln Lys Gln Pro Thr Ile Phe
1               5                   10                  15

Gln Asn Lys Lys Arg Val Leu Leu Gly
            20                  25

<210>  25
<211>  31
<212>  PRT
<213>  Homo sapiens

<400>  25

Ser Ala Ser Ala Leu Gly Gly Val Lys Val Glu Arg Gln Val Phe Gly
1               5                   10                  15

Glu Ala Thr Lys Gln Pro Gly Ile Thr Phe Ile Ala Ala Lys Phe
            20                  25                  30

<210>  26
<211>  21
<212>  PRT
<213>  Homo sapiens

<400>  26

Gly Gly Val Lys Val Glu Arg Gln Val Phe Gly Glu Ala Thr Lys Gln
1               5                   10                  15

Pro Gly Ile Thr Phe
            20

<210>  27
<211>  79
<212>  PRT
<213>  Homo sapiens

<400>  27

<210>  28
<211>  78
<212>  PRT
<213>  Homo sapiens

<400>  28

Ser His Lys Gln Ile Tyr Tyr Ser Asp Lys Tyr Asp Asp Glu Glu Phe
1               5                   10                  15

Glu Tyr Arg His Val Met Leu Pro Lys Asp Ile Ala Lys Leu Val Pro
            20                  25                  30

Lys Thr His Leu Met Ser Glu Ser Glu Trp Arg Asn Leu Gly Val Gln
        35                  40                  45

Gln Ser Gln Gly Trp Val His Tyr Met Ile His Glu Pro Glu Pro His
    50                  55                  60

Ile Leu Leu Phe Arg Arg Pro Leu Pro Lys Lys Pro Lys Lys
65                  70                  75

<210>  29
<211>  41
<212>  PRT
<213>  Homo sapiens

<400>  29

Ser Ala Ser Ala Leu Gly Gly Val Lys Val Glu Arg Gln Val Phe Gly
1               5                   10                  15

Glu Ala Thr Lys Gln Pro Gly Ile Thr Phe Ile Ala Ala Lys Phe Asp
            20                  25                  30

Gly Ile Leu Gly Met Ala Tyr Pro Arg
        35                  40

<210>  30
<211>  31
<212>  PRT
<213>  Homo sapiens

<400>  30

Gly Gly Val Lys Val Glu Arg Gln Val Phe Gly Glu Ala Thr Lys Gln
1               5                   10                  15

Pro Gly Ile Thr Phe Ile Ala Ala Lys Phe Asp Gly Ile Leu Gly
            20                  25                  30

<210>  31
<211>  103
<212>  PRT
<213>  Homo sapiens

<400>  31

Ala Met Arg Ser Met Ala Ser Gly Gly Gly Val Pro Thr Asp Glu Glu
1               5                   10                  15

Gln Ala Thr Gly Leu Glu Arg Glu Ile Met Leu Ala Ala Lys Lys Gly
            20                  25                  30

Leu Asp Pro Tyr Asn Val Leu Ala Pro Lys Gly Ala Ser Gly Thr Arg
        35                  40                  45

Glu Asp Pro Asn Leu Val Pro Ser Ile Ser Asn Lys Arg Ile Val Gly
    50                  55                  60

Cys Ile Cys Glu Glu Asp Asn Thr Ser Val Val Trp Phe Trp Leu His
65                  70                  75                  80

Lys Gly Glu Ala Gln Arg Cys Pro Arg Cys Gly Ala His Tyr Lys Leu
                85                  90                  95

Val Pro Gln Gln Leu Ala His
            100

<210>  32
<211>  98
<212>  PRT
<213>  Homo sapiens

<400>  32

Ala Ser Gly Gly Gly Val Pro Thr Asp Glu Glu Gln Ala Thr Gly Leu
1               5                   10                  15

Glu Arg Glu Ile Met Leu Ala Ala Lys Lys Gly Leu Asp Pro Tyr Asn
            20                  25                  30

Val Leu Ala Pro Lys Gly Ala Ser Gly Thr Arg Glu Asp Pro Asn Leu
        35                  40                  45

Val Pro Ser Ile Ser Asn Lys Arg Ile Val Gly Cys Ile Cys Glu Glu
    50                  55                  60

Asp Asn Thr Ser Val Val Trp Phe Trp Leu His Lys Gly Glu Ala Gln
65                  70                  75                  80

Arg Cys Pro Arg Cys Gly Ala His Tyr Lys Leu Val Pro Gln Gln Leu
                85                  90                  95

Ala His

<210>  33
<211>  20
<212>  PRT
<213>  Homo sapiens

<400>  33

Arg Arg Ile Val Asp Gly Lys Val Val Ser Glu Thr Asn Asp Thr Lys
1               5                   10                  15

Val Leu Arg His
            20

<210>  34
<211>  15
<212>  PRT
<213>  Homo sapiens

<400>  34

Gly Lys Val Val Ser Glu Thr Asn Asp Thr Lys Val Leu Arg His
1               5                   10                  15


<210>  35
<211>  31
<212>  PRT
<213>  Homo sapiens

<400>  35

Ala Glu Val Glu Gln Lys Lys Lys Arg Thr Phe Arg Lys Phe Thr Tyr
1               5                   10                  15


Arg Gly Val Asp Leu Asp Gln Leu Leu Asp Met Ser Tyr Glu Gln
            20                  25                  30


<210>  36
<211>  26
<212>  PRT
<213>  Homo sapiens

<400>  36

Ala Glu Val Glu Gln Lys Lys Lys Arg Thr Phe Arg Lys Phe Thr Tyr
1               5                   10                  15


Arg Gly Val Asp Leu Asp Gln Leu Leu Asp
            20                  25


<210>  37
<211>  104
<212>  PRT
<213>  Homo sapiens

<400>  37

Ser Gly Leu Glu Ala Ile Gln Arg Thr Pro Lys Ile Gln Val Tyr Ser
1               5                   10                  15


Arg His Pro Ala Glu Asn Gly Lys Ser Asn Phe Leu Asn Cys Tyr Val
            20                  25                  30


Ser Gly Phe His Pro Ser Asp Ile Glu Val Asp Leu Leu Lys Asn Gly
        35                  40                  45


Glu Arg Ile Glu Lys Val Glu His Ser Asp Leu Ser Phe Ser Lys Asp
    50                  55                  60

Trp Ser Phe Tyr Leu Leu Tyr Tyr Thr Glu Phe Thr Pro Thr Glu Lys
65                  70                  75                  80

Asp Glu Tyr Ala Cys Arg Val Asn His Val Thr Leu Ser Gln Pro Lys
                85                  90                  95

Ile Val Lys Trp Asp Arg Asp Met
            100

<210>  38
<211>  99
<212>  PRT
<213>  Homo sapiens

<400>  38

<210>  39
<211>  24
<212>  PRT
<213>  Homo sapiens

<400>  39

Ala Pro Ala Ser Ala Leu Val Arg Ile Pro Leu His Lys Phe Thr Ser
1               5                   10                  15

Ile Arg Arg Thr Met Ser Glu Val
            20

<210>  40
<211>  14
<212>  PRT

<213>  Homo sapiens

<400>  40

Leu Val Arg Ile Pro Leu His Lys Phe Thr Ser Ile Arg Arg
1               5                   10

<210>  41
<211>  30
<212>  PRT
<213>  Homo sapiens

<400>  41

His Phe Val Lys Leu Ile Ser Trp Tyr Asp Asn Glu Phe Gly Tyr Ser
1               5                   10                  15

Asn Arg Val Val Asp Leu Met Ala His Met Ala Ser Lys Glu
            20                  25                  30

<210>  42
<211>  25
<212>  PRT
<213>  Homo sapiens

<400>  42

Ile Ser Trp Tyr Asp Asn Glu Phe Gly Tyr Ser Asn Arg Val Val Asp
1               5                   10                  15

Leu Met Ala His Met Ala Ser Lys Glu
            20                  25

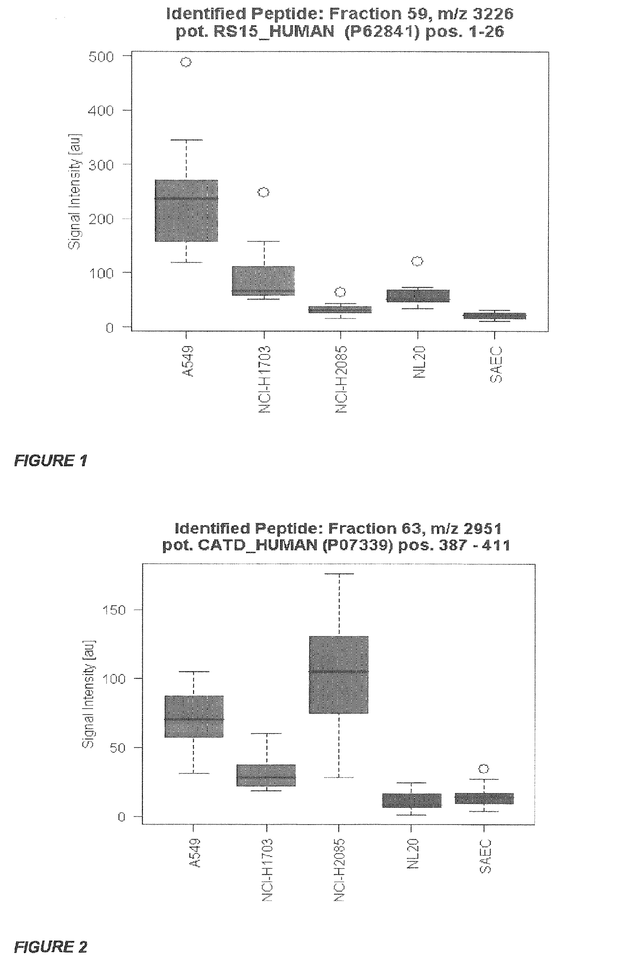

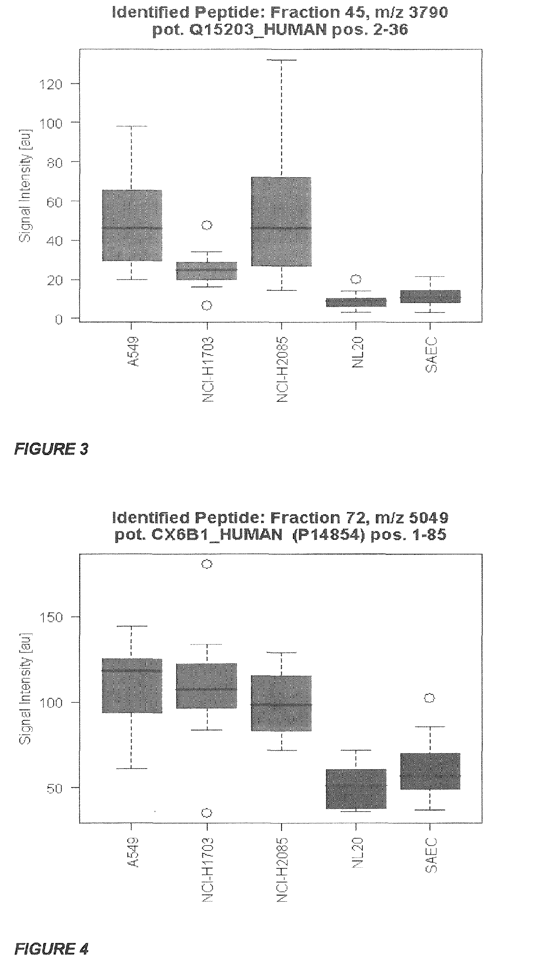

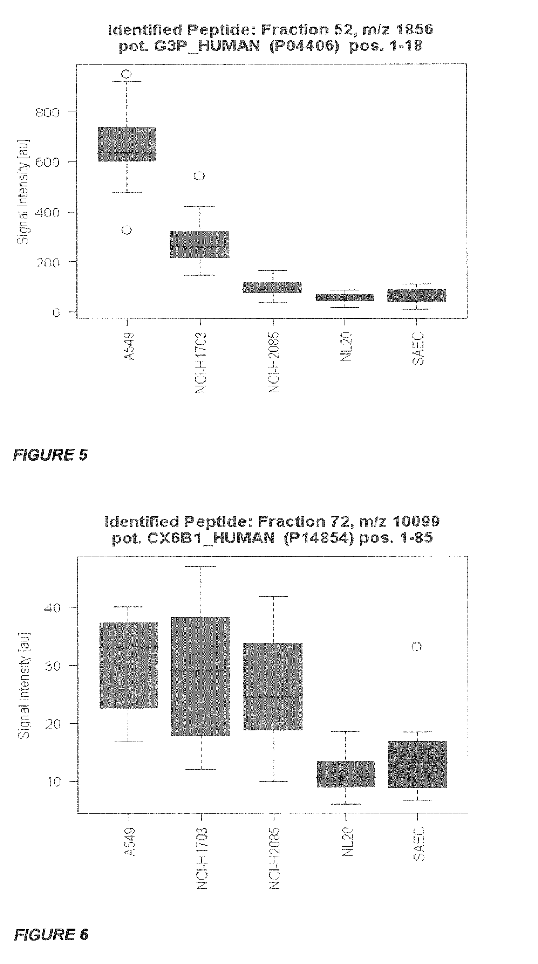

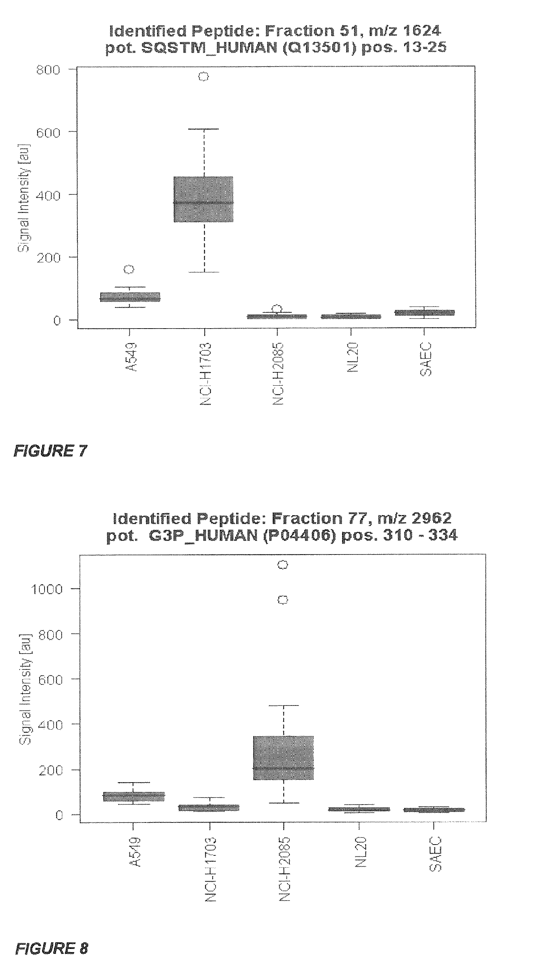

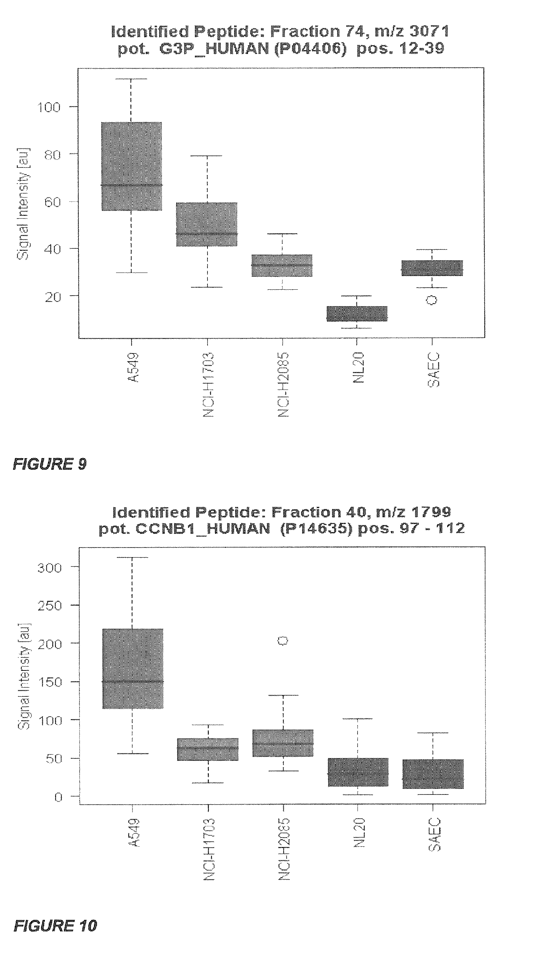

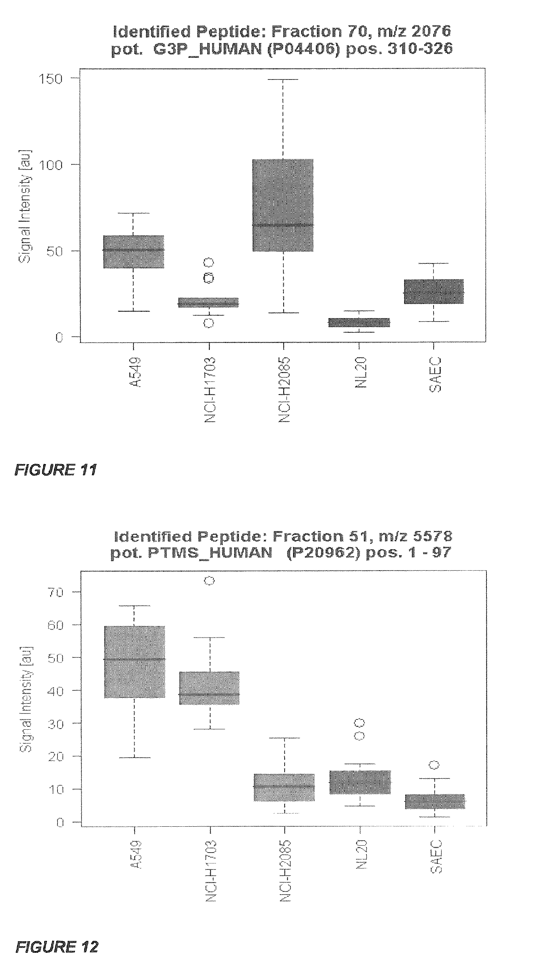

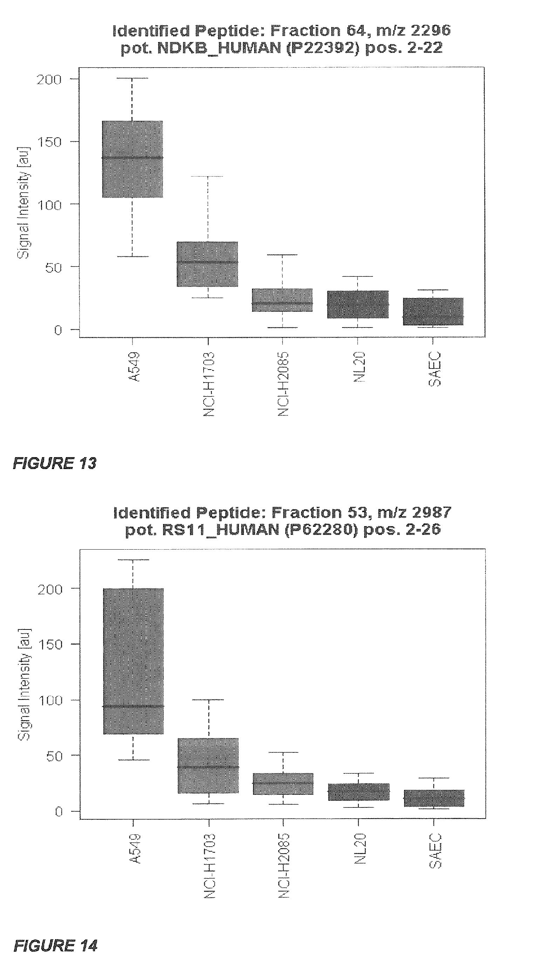

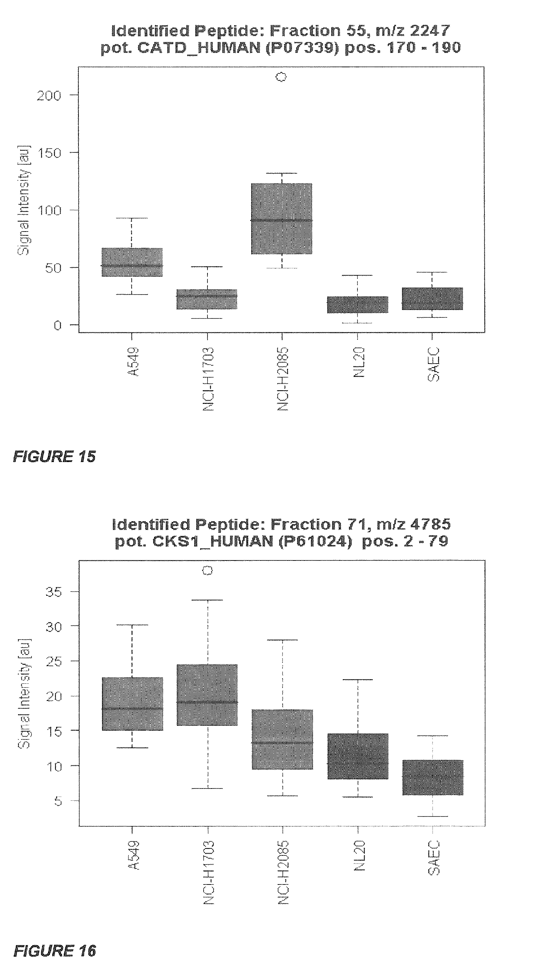

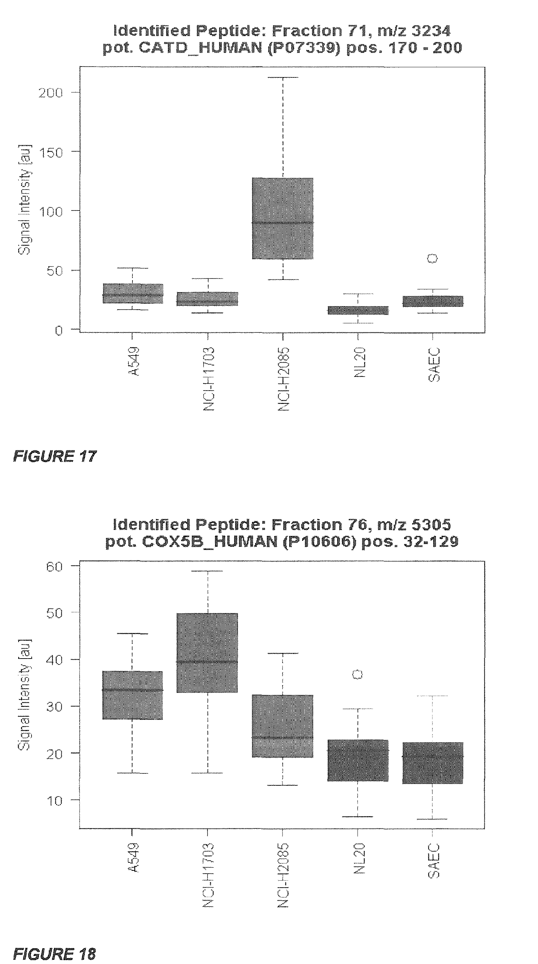

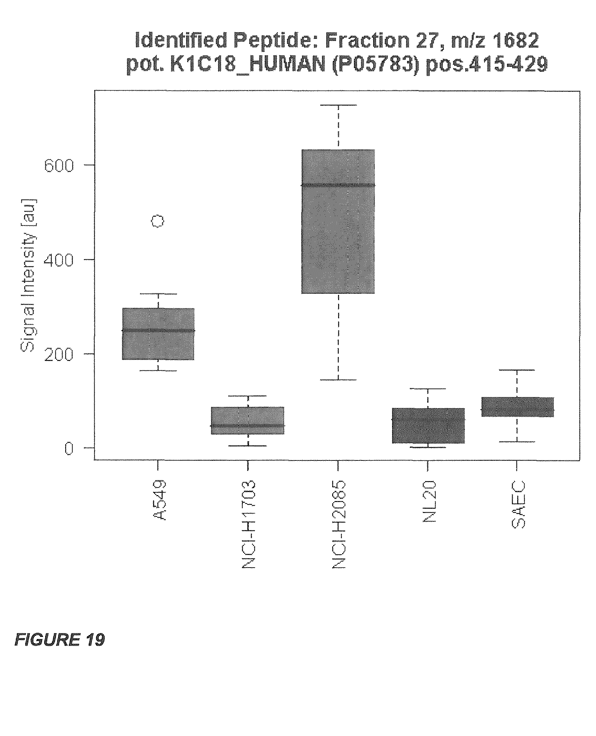

## Description

The present invention relates to a method for the detection of proteins and peptides associated with cancerous cells in lung tissue samples. More specifically, the invention relates to a method of detecting significant changes in the amount and/or chemical structure of peptides and/or proteins associated with lung tumor cells. The present invention further provides a method for early diagnosis and prognosis of lung cancer and for assessing the risk of getting lung cancer.

Smoking enhances the risk of getting lung cancer. The survival rate in lung cancer is poor and did not show any significant increase in the past despite of the increase in general medical knowledge. For most tumors there is a strong correlation between the survival rate and the time point of disease diagnosis, because anti-cancer therapies work best at the early stages of the disease, when there are only a few malignant or pre-malignant cells present. Therefore, sensitive methods for testing are required. The use of molecular markers which are somehow indicative and thus diagnostic for cancerous cells is one of the most promising ways to achieve this goal.

Cancer cells evolve from formerly normal cells. Throughout the transformation process they pass different stages. A couple of changes on the molecular level accompany this transformation process. Knowledge of such subtle molecular differences allows for a better diagnosis at early stages of the disease. It may also allow for a risk determination of getting the disease by the detection of molecules indicative for pre-malignant cells which have a higher incidence of transforming into tumor cells but may also stay pre-malignant.

The common methods used in the art for the detection of lung cancer are mainly based on imaging the lung via x-rays, nuclear magnetic resonance (NMR), or positron emission tomography (PET scan). These methods used in the art for detection of lung cancer show a series of disadvantages, e.g. are insensitive and make results difficult to interpret. X-ray imaging is only capable of detecting tumors >2 mm (spiral computer tomography) which represents a late stage in tumorigenesis, where the tumor is already growing invasively and spreading metastasis and thus difficult to treat. X-rays are known to induce tumors themselves, which restrict its use as a screening method for lager populations and is mainly used for people exhibiting already symptoms of lung cancer. An alternative method, nuclear resonance imaging (NMR) has a similar sensitivity. Although there is no exposure to harmful radiation, the high costs and time needed for NMR-imaging are preventing NMR imaging from being a screening tool for larger populations.

Thus, a method which allows for a higher sensitivity and standardization of the results and is capable of screening large populations for detection of lung cancer would be desirable.

Within the scope of the present invention it could be shown that biomarkers molecules such as proteins and peptides indicative for tumor cells can be used for the detection of cancerous cells in tissue samples thus leading to less harmful, more sensitive and more cost effective diagnosis of lung cancer compared to the current NMR and x-ray imaging techniques. Said biomarker molecules may provide an enhanced diagnostic value and/or can be used for the assessment of the risk of getting lung cancer.

The present invention now provides polypeptides, proteins, including precursor proteins incorporating said polypeptides, and derivatives or variants thereof which are differentially expressed in proliferating cells, particularly in lung cancerous cells, more particularly in cells of non-small cell lung cancer such as, for example, in lung adenocarcinoma cells, squamous cells or several types of large cells, as compared to non-proliferating cells, particularly pre-cancerous or non-cancerous natural cells. This differential expression results in an alteration in said cancerous cells of (a) the amount or (b) the expression pattern of said polypeptides or proteins; or (c) the extent of chemical modifications occurring in said polypeptides or proteins. These alterations, which may be quantitative or qualitative in nature, are specific for the respective cell types or the transformation stage and thus allow to discriminate between proliferating cancerous cells, particularly proliferating lung cancer cells, more particularly cells of non-small cell lung cancer such as, for example, lung adenocarcinoma cells, squamous cells or several types of large cells, and non-proliferating cells, particularly pre-cancerous or non-cancerous natural cells.

The present invention further provides polynucleotides comprising a nucleotide sequence encoding a polypeptide, protein, or precursor proteins according to the present invention as discloses herein in the various embodiments, but particularly a polypeptide, protein, or precursor protein comprising said polypeptide, selected from the group of polypeptides exhibiting an amino acid sequence as shown in SEQ ID NOs: 1-42, or a fragment thereof.

A quantitative alteration may be demonstrated as a decrease or an increase, particularly an increase, of the polypeptide or protein level in the proliferating cell or of the amount of chemical modifications which take place in the proliferating cell, particularly in proliferating cancerous cells, particularly proliferating lung cancer cells, more particularly in cells of non-small cell lung cancer such as, for example, in lung adenocarcinoma cells, squamous cells or several types of large cells.

A qualitative alteration may be demonstrated as a change in the expression pattern or profile of a defined panel of polypeptides or proteins or in the kinds of chemical modifications of said polypeptide or proteins that can be observed in proliferating cells, particularly in proliferating cancerous cells, particularly in proliferating lung cancer cells, more particularly in cells of non-small cell lung cancer such as, for example, in lung adenocarcinoma cells, squamous cells or several types of large cells, as compared to non-proliferating cells, particularly pre-cancerous or non-cancerous natural cells.

In particular, the present invention provides a panel of polypeptides according to the present invention and as disclosed herein in the various embodiments that can be used as a biomarker for the detection of proliferating cancerous cells, particularly of proliferating lung cancer cells, more particularly of cells of non-small cell lung cancer such as, for examples, lung adenocarcinoma cells, squamous cells or several types of large cells, comprising a plurality of polypeptides molecules, or fragments thereof, selected from the following group of 14 polypeptides: glycealdehyde-3-phosphate dehydrogenase, cathepsin D, G2/mitochondrial specific cyalin-B1, cytochrome c oxidase polypeptides VIb isoform 1, nucleoside diphosphate kinase B, prothymosin alpha, 40S ribosomal protein S11, cyclin-dependent kinase regulatory subunit 1, mitochondrial cytochrome c oxidase subunit Vb, sequestosome-1, cytokeratin-18, parathymosin, beta-2 microglobulin and 40S ribosomal protein S15.

In particular, the plurality of polypeptide molecules comprises polypeptides each comprising an amino acid sequence selected from the group of sequences as shown in SEQ ID NOs: 1-42.

In particular, the invention provides a sub-set of polypeptides according to the present invention and as disclosed herein in the various embodiments comprising at least 2, particularly at least 3, particularly at least 4, particularly at least 5, particularly at least 6, particularly at least 7, particularly at least 8, particularly at least 9, particularly at least 10, particularly at least 11, particularly at least 12, particularly at least 13, and especially 14 polypeptide molecules as depicted in Table 1 and showing an amino acid sequence as given in SEQ ID NOs: 1-42. Each of said polypeptide molecules or a sub-set thereof is indicative of the presence of cancerous cells, particularly cancerous cells in lung tissue and can thus be used as a molecular biomarkers for the detection of lung cancer, particularly of non-small cell lung cancer such as, for examples, lung adenocarcinoma, squamous cell carcinoma or large cell carcinoma.

In one embodiment, the invention relates to a method of detecting cancerous cells in a tissue sample, preferably a tissue sample isolated from the human or animal body by
a) determining in said sample the level of a biomarker polypeptide or a panel of biomarker polypeptides which are differentially expressed in proliferating cells, particularly in proliferating cancerous cells, particularly in proliferating lung cancer cells, more particularly in cells of non-small cell lung cancer such as, for example, in lung adenocarcinoma cells, squamous cells or several types of large cells, as compared to pre-cancerous and non-cancerous, normal cells, according to the present invention and as disclosed herein in the various embodiments,
b) determining a reference value, and
c) comparing the level of the biomarkers polypeptide or panel of biomarker peptides determined in step a) with the reference value determined in step b).

In a further embodiment, the invention relates to a method of detecting cancerous cells in a tissue sample by
a) determining in said sample the level of a biomarker peptide or a panel of biomarker peptides which are differentially expressed in proliferating cells, particularly in proliferating cancerous cells, particularly in proliferating lung cancer cells, more particularly in cells of non-small cell lung cancer such as, for example, in lung adenocarcinoma cells, squamous cells or several types of large cells, as compared to pre-cancerous and non-cancerous, normal cells, according to the present invention and as disclosed herein in the various embodiments,
b) determining a reference value in a control sample known to contain pre-cancerous or non-cancerous cells using the same biomarkers peptide or panel of biomarker peptides as used in step a), and
c) comparing the level of the biomarker polypeptide or panel of biomarker peptides determined in step a) with the reference value determined in step b).
   wherein any alteration in the level of the biomarker peptide or panel of biomarker peptides in the test sample as compared to the control sample is verified for statistical significance.

In one embodiment, the invention relates to a method of detecting cancerous cells in a sample, preferably a sample isolated from the human or animal body by bronchoalveolar lavage and
a) determining in said sample the level of a biomarker polypeptide or a panel of biomarker polypeptides which are differentially expressed in proliferating cells, particularly in proliferating cancerous cells, particularly in proliferating lung cancer cells, more particularly in cells of non-small cell lung cancer such as, for example, in lung adenocarcinoma cells, squamous cells or several types of large cells, as compared to pre-cancerous and non-cancerous, normal cells, according to the present invention and as disclosed herein in the various embodiments,
b) determining a reference value, and
c) comparing the level of the biomarker polypeptide or panel of biomarker peptides determined in step a) with the reference value determined in step b).

In one embodiment, the invention relates to a method of detecting cancerous cells in a sample, preferably a sample derived from human or animal exhaled breathe condensate by
a) determining in said sample the level of a biomarker polypeptide or a panel of biomarker polypeptides which are differentially expressed in proliferating cells, particularly in proliferating cancerous cells, particularly in proliferating lung cancer cells, more particularly in cells of non-small cell lung cancer such as, for example, in lung adenocarcinoma cells, squamous cells or several types of large cells, as compared to pre-cancerous and non-cancerous, normal cells, according to the present invention and as disclosed herein in the various embodiments,
b) determining a reference value, and
c) comparing the level of the biomarkers polypeptide or panel of biomarker peptides determined in step a) with the reference value determined in step b).

In particular, the invention relates to a method as described herein in the various embodiments, wherein the extent of the alteration between the value determined for the biomarker polypeptide and the reference value amounts to at least 200%, 300%, 400%, 500%, 600%, 700%, 800%, 900%, and 1000%.

In one embodiment, the invention provides a method for diagnosing in an individual a disorder associated with abnormal lung cell proliferation comprising
a) obtaining a sample from said individual to be tested,
b) determining in said sample material the level of a biomarker polypeptide or a panel of biomarkers polypeptides which are differentially expressed in proliferating cells, particularly in proliferating cancerous cells, particularly in proliferating lung cancer cells, more particularly in cells of non-small cell lung cancer such as, for example, in lung adenocarcinoma cells, squamous cells or several types of large cells, as compared to pre-cancerous and non-cancerous, normal cells, according to the present invention and as disclosed herein in the various embodiments,
c) determining a reference value in a control sample known to contain pre-cancerous or non-cancerous cells using the same biomarker peptide or panel of biomarker peptides as used in step b), and
d) comparing the level of the biomarker polypeptide or panel of biomarker peptides determined in step b) with the reference value determined in step c).
wherein any alteration in the level of the biomarkers peptide or panel of biomarker peptides in the test sample as compared to the control sample is verified for statistical significance and the diagnosis is predicted in consideration of the extent of the alteration observed. In particular, the invention relates to a method as described herein in the various embodiments, wherein the extent of the alteration between the value determined for the biomarker polypeptides and the reference value amounts to at least 200%, 300%, 400%, 500%, 600%, 700%, 800%, 900%, and 1000%.

In a specific embodiment of the invention, the sample to be used in any of the previously described methods is a liquid containing polypeptide or fragments thereof, a liquid containing cells or cell debris, particularly a body fluid, e.g. blood, plasma, serum, sputum or a secretion, a tissue sample, particularly a lung tissue sample, a cell sample, particularly a sample of lung cells, or a sample obtained by biopsy, particularly by biopsy of the lung.

In another specific embodiment of the invention, the disorder associated with abnormal cell proliferation recited in any of the previously described methods is a cancer, particularly a lung cancer or a precursor lesion, particularly a precursor lesion of the lung, and more particularly a non-small cell lung cancer such as, for example, a lung adenocarcinoma, a squamous cell carcinoma or a large cell carcinoma.

In one embodiment of the invention, the biomarkers polypeptide or the panel of biomarker polypeptides to be used in any of the methods according to the present invention as described herein in the various embodiments is selected from the group of polypeptides comprising an amino acid sequence as shown in SEQ ID NOs: 1 - 42.

In another specific embodiment of the invention, the panel of biomarkers polypeptides to be used in any of the methods of the invention as described herein in the various embodiments comprises at least two biomarker polypeptide selected from the group of polypeptides comprising an amino acid sequence as shown in SEQ ID NOs: 1 - 42.

In one embodiment, the method of the invention as described herein in the various embodiments may be used for distinguishing and classifying normal and tumor tissues comprising determining in said tissues differential expression of a panel of at least 2, particularly at least 3, particularly at least 4, particularly at least 5, particularly at least 6, particularly at least 7, particularly at least 8, particularly at least 9, particularly at least 10, particularly at least 11, particularly at least 12, particularly at least 13, and especially 74 biomarker, selected from the biomarkers depicted in Table 1.

The invention further relates to one of the previously described methods wherein the biomarkers polypeptide comprises an amino acid sequence exhibiting at least 80%, particularly at least 85%, more particularly at least 90%, 91%, 92%, 93%, 94%, 95%, 96%, 97%, 98% and up to 99**%** sequence identity to an amino acid sequence selected from the group of sequences shown in SEQ ID NOs**:** 1-42.

In still another specific embodiment of the invention, the biomarker polypeptide or the panel of peptides to be used in any of the previously described methods is expressed in proliferating cells, particularly in proliferating cancerous cells such as to result in a higher concentration in cancerous as compared to pre-cancerous and non-cancerous normal cells.

In one aspect, the invention relates to any of the methods described herein in the various embodiments, wherein the level of the biomarker polypeptide or a panel of biomarker polypeptide according to the invention which is associated with proliferating cells, particularly proliferating cancerous cells, is detected in the sample material using a test system for detecting said biomarkers polypeptide.

In one embodiment of the invention, determination of biomarkers values within a method according to the invention as described herein in the various embodiments is accomplishes by performing an *in-vitro* assay, particularly an *in-vitro* assay selected from the group consisting of an antibody-based assay such as an immunoassay, a histological or cytological assay, an expression level assay such as an RNA, expression level assay and an aptamer-based assay.

In certain embodiments of the invention, the level of the biomarkers polypeptides in the sample material may be determined on DNA**,** mRNA and/or protein level. Accordingly, the present invention further relates to a method as described herein in the various embodiments, wherein the level of the biomarker polypeptide or a panel of maker polypeptides according to the invention in said sample material is determined by a nucleic acid hybridisation technique, an immunological method or a proteomics technique.

Immunological methods that may be used within the scope of the invention include, for example, a binding assay, e.g. Enzyme-Linked Immuno Sorbent Assay (ELISA) or modifications thereof where the binding agent is not an antibody.

In a specific embodiment of the invention, an enzyme-linked immunosorbent assay may be performed for determining biomarker values.

The level of expression of a biomarkers gene may be determined by measuring the amount of biomarker messenger RNA, for example, by DNA-DNA hybridization, RNA-DNA hybridization, reverse transcription-polymerase chain reaction (PCR), or real time quantitative PCR; and comparing the results to a reference based on samples from clinically-characterized patients and/or cell lines of a known genotype/phenotype. As an alternative to amplification techniques, hybridization assays can be performed. Microarray-based assays can be used to detect and quantify the amount of biomarker gene transcript using DNA- or oligonucleotide-based arrays. Microarray technology allows multiple biomarker gene transcripts and/or samples from different subjects to be analyzed in one reaction. Typically, mRNA isolated from a sample is converted into labeled nucleic acids by reverse transcription and optionally *in vitro* transcription (cDNAs or cRNAs labeled with, for example, Cy3 or Cy5 dyes) and hybridized in parallel to probes present on an array. See, for example, Schulze et al., Nature Cell Biol., 3 (2001), E190; and Klein et al., J Exp Med, 2001, 1625-1638, which are incorporated herein by reference in their entirety. Standard Northern analyses can be performed if a sufficient quantity of the test cells can be obtained. Utilising such techniques, quantitative as well as size related differences between biomarkers transcripts can also be detected.

The present invention provides isolated biomarker polynucleotides or variants thereof, which can be used, for example, as hybridization probes or primers ("biomarker probes" or "biomarker primers") to detect or amplify nucleic acids encoding a biomarker polypeptides. In a specific embodiment of the invention, said biomarkers polynucleotides or variants thereof encodes a polypeptide, protein, or precursor proteins according to the present invention as disclosed herein in the various embodiments, but particularly a polypeptide, protein, or precursor protein comprising a polypeptide selected from the group of polypeptides exhibiting an amino acid sequence as shown in SEQ ID NOs: 1-42, or a fragment thereof. The term "biomarker gene product" encompasses both mRNA as well as translated polypeptide as a gene product of a biomarkers. The present invention also provides "biomarker antibodies" that immunospecifically binds to the respective biomarker proteins or polypeptides. Compositions comprising labeled biomarker polynuleotides, or labeled biomarker antibodies are also encompassed by the invention.

The invention also provides compositions comprising biomarker polynucleotides, biomarkers polypeptides, or biomarker antibodies according to the present invention as disclosed herein in the various embodiments. The invention further provides diagnostic reagents for use in the methods of the invention, such as but not limited to reagents for flow cytometry and/or immunoassays that comprise fluorochrome-labeled antibodies that bind to one of the biomarker polypeptides

Nucleic acid molecules comprising nucleic acid sequences encoding the biomarker polypeptides or proteins of the invention, or genomic nucleic acid sequences from the biomarkers genes (e.g., intro sequences, 5' and 3' non-translated sequences), or their complements thereof (i.e., antisense polynucleotides), are collectively referred to as "biomarker genes", "biomarker polynucleotides" or "biomarker nucleic acid sequences" of the invention. The present invention also provides isolated biomarker polynucleotide or variants thereof, which can be used, for example, as hybridization probes or primers ("biomarker probes" or "biomarker primers") to detect or amplify nucleic acids encoding a polypeptide of the invention. The present invention also provides "biomarker antibodies", including polyclonal, monoclonal, or recombinant antibodies, and fragments and variants thereof, that immunospecifically binds the respective biomarkers proteins as listed in Table 1. Compositions comprising labeled biomarker polynucleotides, and labeled biomarkers antibodies to the biomarkers proteins or polypeptides are also encompassed by the invention.

An isolated biomarker polynucleotide can comprise flanking sequences (*i:e*:, sequences located at the 5' or 3' ends of the nucleic acid), which naturally flank the nucleic acid sequence in the genomic DNA of the organism from which the nucleic acid is derived. However, an isolated polynucleotide does not include an isolated chromosome, and does not include the poly(A) tail of an mRNA, if present. For example, in various embodiments, the isolated biomarker polynucleotide can comprise less than about 5 kb, 4 kb, 3 kb, 2 kb, 1 kb, 0.5 kb or 0.1 kb of nucleotide sequences which naturally flank the coding sequence in genomic DNA of the cell from which the nucleic acid is derived. In other embodiments, the isolated biomarker polynucleotide is about 10-20, 21-50, 51-100, 101-200, 201-400, 401-750, 751-1000, 1001-1500 bases in length.

In various embodiments, the biomarkers polynucleotides and/or their flanking sequences of the invention as disclosed herein in the various embodiments are used as molecular probes in hybridization reactions or as molecular primers in nucleic acid extension reactions. In these instances, the biomarkers polynucleotide may be referred to as biomarker probes and biomarker primers, respectively, and the biomarkers polynucleotides present in a sample which are to be detected and/or quantified are referred to as target biomarker polynucleotides. Two biomarker primers are commonly used in DNA amplification reactions and they are referred to as biomarkers forward primer and biomarker reverse primer depending on their 5' to 3' orientation relative to the direction of transcription. A biomarker probe or a biomarker primer is typically an oligonucleotide which binds through complementary base pairing to a subsequence of a target biomarkers polynudlotide. The biomarkers probe may be, for example, a DNA fragment prepared by amplification methods such as by PCR or it may be chemically synthesized. A double stranded fragment may then be obtained, if desired, by annealing the chemically synthesized single strands together under appropriate conditions or by synthesizing the complementary strand using DNA polymerase with an appropriate primer. Where a specific nucleic acid sequence is given, it is understood that the complementary strand is also identified and included as the complementary strand will work equally well in situations where the target is a double stranded nucleic acid. A nucleic acid probe is complementary to a target nucleic acid when it will annual only to a single desired position on that target nucleic acid under proper annealing conditions which depend, for example, upon a probe's length, base composition, and the number of mismatches and their position on the probe, and must often be determined empirically. Such conditions can be determined by those of skill in the art.

In one embodiment of the invention, a test system is provided to be used in one of the methods according to the invention and as described herein in the various embodiments comprising a biomarker polypeptides and an agent capable of specifically binding to the biomarkers polypeptide and a reagent capable of detecting this specific binding of the biomarker polypeptide to the binding agent.

In one embodiment, the binding agent is an antibody or a binding fragment thereof, or a receptor which binds to an epitope of the biomarker polypeptides according to the present invention.

Various chemical or biochemical derivatives of the antibodies or antibody fragments can be produced using known methods. One type of derivative which is diagnostically useful is an immunoconjugate comprising an antibody molecule, or an antigen-binding fragment thereof, to which is conjugated a detectable label. However, in many embodiments, the biomarker antibody is not labeled but in the course of an assay, it becomes indirectly labeled by binding to or being bound by another molecules that is labeled. The invention encompasses molecular complexes comprising a biomarker antibody and a label, as well as immunocomplexes comprising a biomarker polypeptide, a biomarker antibody, and immunocomplexes comprising a biomarker polypeptide, a biomarkers antibody, and a label.

Examples of detectable substances include various enzymes, prosthetic groups, fluorescent materials, luminescent materials, bioluminescent materials, and radioactive materials. Examples of suitable enzymes include horseradish peroxidase, alkaline phosphatase, p-galactosidase, or acetylcholinesterase; examples of suitable prosthetic group complexes include streptavidin/biotin and avidin/biotin; examples of suitable fluorescent materials, include umbelliforones, fluoresceins, fluorescein isothiocyanate, rhodamines, dichlorotriazinylamine fluorescein, dansyl chloride, phycoerythrins, Alexa Fluor 647, Alexa Fluor 680, DilC₁₉(3), Rhodamine Red-X, Alexa Fluor 660, Alexa Fluor 546, Texas Red, YOYO-1 + DNA, tetramethylrhodamine, Alexa Fluor 594, BODIPY FL, Alexa Fluor 488, Fluorescein, BODIPY TR, BODIPY TMR, carboxy SNARF-1, FM 1-43, Fura-2, Indo-1, Cascade Blue, NO, DAPI, Alexa Fluor 350, aminomethylcoumarin, Lucifer yellow, Propidium iodide, or dansylamide; an example of a luminescent material includes luminol, examples of bioluminescent materials include green fluorescent proteins, modified green fluorescent proteins, luciferase, luciferin, and aequorin, and examples of suitable radioactive materials include ¹²⁵I, ¹³¹I, ³⁵S or 3H.

Immunoassays for biomarkers polypeptides will typically comprise incubating a sample, such as a biological fluid, a tissue extract, freshly harvested cells, or lysates of cells, in the presence of a detestably labeled antibody capable of identifying biomarkers gene products or conserved variants or peptide fragments thereof, and detecting the bound antibody by any of a number of techniques well-known in the art. One way of measuring the level of biomarkers polypeptides with a specific biomarker antibody of the present invention is by enzyme immunoassay (EIA) such as an enzyme-linked immunosorbent assay (ELISA) (Voller, A. et al., J. Clip. Pathol. 31:507-520 (1978); Butler, J.E., Moth. Enzymol. 73:482-523 (1981); Maggio, E. (ed.), Enzyme Immunoassay, CRC Press, Boca Raton, FL, 1980). The enzyme, either conjugated to the antibody or to a binding partner for the antibody, when later exposed to an appropriate substrate, will react with the substrate in such a manner as to produce a chemical moiety which can be detected, for example, by spectrophotometric, or fluorimetric means.

The biological sample may be brought in contact with and immobilized onto a solid phase support or carrier such as nitrocellulose, or other solid support which is capable of immobilizing cells, cell particles or soluble proteins. The support may then be washed with suitable buffers followed by treatment with the detectably labeled biomarker antibody. The solid phase support may then be washed with the buffer a second time to remove unbound antibody. The amount of bound label on solid support may then be detected by conventional means. A well known example of such a technique is Western blotting.

In certain embodiments of the invention, the biomarker value is determined by performing mass spectrometry.

In further embodiments of the invention, a microarray-based immunohistochemical analysis or surface enhanced laser desorption/ionization may be performed for determining biomarker values.

Another aspect of the present invention relates to a testing kit for performing the detection of polypeptides according to the present invention or precursor proteins thereof. The kit may be for example a diagnostic kit or a research kit.

In one embodiment of the present invention, the kit incorporating the biomarker polypeptide according to the invention and described herein in the various embodiments is used for the diagnosis of lung cancer.

In another aspect of the invention, a kit is provided comprising at least an agent suitable for detecting the biomarkers molecules disclosed herein. Furthermore a kit according to present invention may comprise:
a) one or more specific antibodies or nucleic acids suitable for specifically binding one or more of the molecules disclosed herein.
b) reagents used in nucleic acid amplification reaction comprising one or more polymerase enzymes, substrates for the amplification reaction, buffers and/or activators
c) compounds for use in the conditioning of samples comprising (magnetic) beads, devices for chromatography, ready to use tubes and/or filters and/or membranes.
d) reagents and buffers suitable for the preparation of polypeptide extracts from given sample tissues and/or stool and/or blood and/or sputum.
e) packings of said kit according to the present invention suitable for use in automated pipetting systems.

These and other objects, features and advantages of the present invention will become apparent after a review of the following detailed description of the disclosed embodiment and the appended claims.

The terms "a", "an" and "the" as used herein are defined to mean "one or more" and include the plural unless the context is inappropriate.

The term "non-small cell lung cancer" as used herein refers to cancers that have different kinds of cancer cells, each type of which grow and spread differently. The types of non-small cell lung cancer are named for the kinds of cells found in the cancer and how the cells look under the microscope.

Squamous cell carcinoma: Cancer that begins in squamous cells, which are thin, flat cells that look like fish scales. This is also called epidermoid carcinoma.

Large cell carcinoma: Cancer that may begin in several types of large cells.

Adenocarcinoma: Cancer that begins in the cells that line the alveoli and make substances such as mucus.

Other less common types of non-small cell lung cancer are: pleomorphic, carcinoid tumor, salivary gland carcinoma, and unclassified carcinoma.

The terms "polypeptide", "peptide", and "protein,", as used herein, are interchangeable and are defined to mean a biomolecule or a fragment thereof composed of amino acids, whether isolated from natural sources, produced by recombinant techniques or chemically synthesized. Polypeptides of the invention typically comprise at least about 6 amino acids.

The term "nucleic acid" or "polynucleotide" refers to a deoxyribonucleotide or ribonucleotide polymer in either single- or double-stranded form, and unless otherwise limited, encompasses known analogs of natural nucleotides that hybridize to nucleic acids in a manner similar to naturally-occurring nucleotides.

The terms "detecting" or "detected" as used herein mean using known techniques for detection of biologic molecules such as immunochemical or histological methods and refer to qualitatively or quantitatively determining the presence or concentration of the biomolecule under investigation.

The terms "antibody" or "antibodies" as used herein is an art recognized term and is understood to refer to molecules or active fragments of molecules that bind to known antigens, particularly to immunoglobulin molecules and to immunologically active portions of immunoglobulin molecules, i.e molecules that contain a binding site that immunospecifically binds an antigen. The immunoglobulin according to the invention can be of any type (IgG, IgM, IgD, IgE, IgA and IgY) or class (IgG1, IgG2, IgG3, IgG4, IgA1 and IgA2) or subclasses of immunoglobulin molecule.

"Antibodies" are intended within the scope of the present invention to include monoclonal antibodies, polyclonal, chimeric, single chain, bispecific, simianized, human and humanized antibodies as well as active fragments thereof. Examples of active fragments of molecules that bind to known antigens include separated light and heavy chains, Fab, Fab/c, Fv, Fab', and **F(ab')₂** fragments, including the products of an Fab immunoglobulin expression library and epitope-binding fragments of any of the antibodies and fragments mentioned above.

A polypeptides "variant" or "derivative", as used herein, is a polypeptide that differs from a native protein in one or more substitutions, deletions, additions and/or insertions, such that the immunogenicity of the polypeptides is not substantially changed. In other words, the ability of a variant to react with antigen-specific antisera may be enhanced or unchanged, relative to the native protein, or may be diminished by less than 50%, and preferably less than 20%, relative to the native protein. Such variants may generally be identified by modifying one of the above polypeptide sequences and devaluating the reactivity of the modified polypeptides with antigen-specific antibodies or antisera as described herein. Preferred variants include those in which one or more portions, such as an N-terminal leader sequence, have been removed. Other preferred variants include variants in which a small portion (e.g., 1-30 amino acids, preferably 5-15 amino acids) has been removed from the N- and/or C-terminal of the mature protein. Polypeptide variants may exhibit at least about 70%, particularly at least about 80%, more particularly at least about 85%, even more particularly at least about 90%, but especially at least about 95% and up to 98% and 99% identity (determined as described above) to the identified polypeptides.

Preferably, a variant contains conservative substitutions. As used herein a "conservative change" refers to alterations that are substantially conformationally or antigenically neutral, producing minimal changes in the tertiary structure of the mutant polypeptides, or producing minimal changes in the antigenic determinants of the mutant polypeptides, respectively, as compared to the native protein. Those of ordinary skill in the art will be able to predict which amino acid substitutions can be made while maintaining a high probability of being conformationally and antigenically neutral. Such guidance is provided, for example in Berzofsky, (1985) Science 229:932-940 and Bowie et al. (1990) Science 247:1306-1310. Factors to be considered that affect the probability of maintaining conformational and antigenic neutrality include, but are not limited to: (a) substitution of hydrophobic amino acids is less likely to affect antigenicity because hydrophobic residues are more likely to be located in a protein's interior; (b) substitution of physiochemically similar, amino acids is less likely to affect conformation because the substituted amino acid structurally mimics the native amino acid; and (c) alteration of evolutionarily conserved sequences is likely to adversely affect conformation as such conservation suggests that the amino acid sequences may have functional importance. One of ordinary skill in the art will be able to assess alterations in protein conformation using well-known assays, such as, but not limited to microcomplement fixation methods and through binding studies using conformation-dependent monoclonal antibodies.

"Sequence Homology or Sequence identity" is used herein interchangeably. The terms "identical" or percent "identity," in the context of two or more nucleic acid or protein sequences, refer to two or more sequences or subsequences that are the same or have a specified percentage of amino acid residues or nucleotides that are the same, when compared and aligned for maximum correspondence, as measured using one of the following sequence comparison algorithms or by visual inspection. If two sequences which are to be compared with each other differ in length, sequence identity preferably relates to the percentage of the nucleotide residues of the shorter sequence which are identical with the nucleotide residues of the longer sequence. Sequence identity can be determined conventionally with the use of computer programs such as the Bestfit program (Wisconsin Sequence Analysis Package, Version 8 for Unix, Genetics Computer Group, University Research Park, 575 Science Drive Madison, WI 53711). Bestfit utilizes the local homology algorithm of Smith and Waterman, Advances in Applied Mathematics 2 (1981), 482-489, in order to find the segment having the highest sequence identity between two sequences. When using Bestfit or another sequence alignment program to determine whether a particular sequence has for instance 95% identity with a reference sequence of the present invention, the parameters are preferably so adjusted that the percentage of identity is calculated over the entire lengfih of the reference sequence and that homology gaps of up to 5% of the total number of the nucleotides in the reference sequence are permitted. When using Bestfit, the so-called optional parameters are preferably left at their preset ("dafault") values. The deviations appearing in the comparison between a given sequence and the above-described sequences of the invention may be caused for instance by addition, deletion, substitution, insertion or recombination. Such a sequence comparison can preferably also be carried out with the program "fasta20u66" (version 2.0u66, September 1998 by William R. Pearson and the University of Virginia; see also W.R. Pearson (1990), Methods in Enzymology 183, 63-98, appended examples and http://workbench.sdsc.edu/). For this purpose, the "default" parameter settings may be used.

Another indication that two nucleic acid sequences are substantially identical is that the two molecules hybridize to each other under stringent conditions. The phrase: "hybridizing specifically to" refers to the binding, duplexing, or hybridizing of a molecule only to a particular nucleotide sequence under stringent conditions when that sequence is present in a complex mixture (e.g., total cellular) DNA or RNA. "Bind(s) substantially" refers to complementary hybridization between a probe nucleic acid and a target nucleic acid and embraces minor mismatches that can be accommodated by reducing the stringency of the hybridization media to achieve the desired detection of the target nucleic acid sequence.

The term "hybridize" as used herein refers to conventional hybridization conditions, preferably to hybridization conditions at which 5xSSPE, 1 % SDS, 1xDenhardts solution is used as a solution and/or hybridization temperatures are between 35°C and 70°C, preferably 65°C. After hybridization, washing is preferably carried out first with 2xSSC, 1% SDS and subsequently with 0.2xSSC at temperatures between 35°C and 75°C, particularly between 45°C and 65°C, but especially at 59°C (regarding the definition of SSPE, SSC and Denhardts solution see Sambrook et al. loc. cit.). High stringency hybridization conditions as for instance described in Sambrook et al, supra, are particularly preferred. Particularly preferred stringent hybridization conditions are for instance present if hybridization and washing occur at 65°C as indicated above. Non-stringent hybridization conditions for instance with hybridization and washing carried out at 45°C are less preferred and at 35°C even less.

Within the scope of the present invention some polypeptides are identified, the expression of which is associated with lung tumor cell lines. In one aspect of the invention, a method is provided which allows to distinguish tumor cells from pre-cancerous and normal cells e.g., in lung cancer, to predict the outcome of the disease and/or to follow up the disease after initial therapy based on the amount of polypeptides detected in said cells or tissues.

In another aspect of the present invention, methods are provided which allow for early detection of cell proliferative disorders such as e.g. lung tumors and for the risk assessment of pre-cancerous cells of becoming tumor cells.

The present invention provides tumor cell associated polypeptides and modified polypeptides, which are differentially expressed in proliferating cells, particularly proliferating cancerous cells of the lung and can therefore be used as biomarkers for the detection of proliferating cancerous cells, Said polypeptide incorporate the sequences given in SEQ ID NOs: 1-42 and in Table 1. As a result of this differential expression, the biomarkers polypeptide are present in the cancerous cells at an altered, but particularly at a higher level than in non-cancerous normal cells. Determination of the biomarker peptide level in a sample obtained from an individual to be tested and comparison with a reference value obtained from a control sample thus allows to distinguish tumor cells from pre-cancerous and normal cells e.g., in lung cancer, to make predictions on the status of the disease and/or to follow up the disease after initial therapy based on the amount of polypeptide detected in said cells or tissues.

Polypeptides that can be used in the present invention as biomarkers molecules in the methods as described herein before may comprise the polypeptides of SEQ ID NOs: 1-42, including modified polypeptides and precursor proteins incorporating said polypeptide sequences.

A biomarkers polypeptide of the invention can comprise a variant of a native protein or polypeptides. A polypeptides "variant," as used herein, is a polypeptides that differs from a native protein in one or more substitutions, deletions, additions and/or insertions, such that the immunogenicity of the polypeptides is not substantially diminished, in particular immunogenicity is diminished by less than 50%, and preferably less than 20%, relative to the native protein. Other preferred variants include variants in which a small portion (e.g., 1-30 amino acids, preferably 5-15 amino acids) has been removed from the N- and/or C-terminal of the mature protein. Polypeptide variants preferably exhibit at least about 70%, more preferably at least about 90% and most preferably at least about 95% identities (determined as described above) to the identified polypeptides.

Preferably, a variant contains conservative substitutions. A "conservative substitution" is one in which an amino acid is substituted for another amino acid that has similar properties, such that one skilled in the art of peptide chemistry would expect the secondary structure and hydropathic nature of the polypeptide to be substantially unchanged. Amino acid substitutions may generally be made on the basis of similarity in polarity, charge, solubility, hydrophobicity, hydrophilicity and/or the amphipathic nature of the residues. For example, negatively charged amino acids include aspartic acid and glutamic acid; positively charged amino acids include lysine and arginine; and amino acids with uncharged polar head groups having similar hydrophilicity values include leucine, isoleucine and valine; glycine and alanine; asparagine and glutamine; and serine, threonine, phenylalanine and tyrosine. Other groups of amino acids that may represent conservative changes include: (1) ala, pro, gly, glu, asp, gln, asn, ser, thr; (2) eyes, ser, tyr, thr; (3) val, ile, leu, met, ala, phe; (4) lys, are, his; and (5) phe, tyr, trp, his, A variant may also, or alternatively, contain nonconservative changes. In a preferred embodiment, variant polypeptides differ from a native sequence by substitution, deletion or addition of five amino acids or fewer. Variants may also (or alternatively) be modified by, for example, the deletion or addition of amino acids that have minimal influence on the immunogenicity, secondary structure and hydropathic nature of the polypeptide.

Precursor proteins are proteins that when subjected to proteolytic cleavage, result in a polypeptides comprising the disclosed polypeptide sequences. In accordance with the present invention, alteration of the amount of a polypeptide or its precursor protein refers to the increase or decrease of the amount of said polypeptides or its precursor protein in proliferating cells, particularly in proliferating cancerous cells as compared to non-proliferating cells, particularly pre-cancerous or non-cancerous natural cells, which is statistically significant, but reaches at least 200%, 300%, 400%, 500%, 600%, 700%, 800%, 900%, and 1000%. The alteration of the amount may comprise for example elevated amounts or reduced amounts of said polypeptide.

Alteration of the amount as used herein may also comprise an alteration in the expression pattern of said polypeptides or precursor proteins of those. E.g. the alteration of the amount may comprise alternative chemical modifications of the polypeptide or its precursor protein. Alteration of the amount of polypeptides may also comprise an increased activity of the mechanisms (e.g., proteolysis) leading to the formation of the polypeptides form large or complex precursor molecules

The level of the biomarker polypeptide in the sample material may be determined on DNA, mRNA and/or protein level. Accordingly, the present invention further relates to a method as described herein before, wherein the level of the biomarker polypeptide or a panel of maker polypeptides according to the invention in said sample material is determined by a nucleic acid hybridisation technique, immunological methods or proteomics technique, and/or mass spectroscopy.

In one embodiment of the invention, a test system is provided to be used in one of the methods according to the invention and as described herein before comprising a biomarker polypeptide and an agent capable of specifically binding to the biomarker polypeptides and a reagent capable of detecting this specific binding of the biomarker polypeptide to the binding agent.

The term binding agent is understood within the scope of this invention to comprise a variety of molecules which are capable of recognizing and binding to specific parts of the biomarker polypeptide molecules including glycopeptides, antibodies, peptidomimetics, nucleic acids, carbohydrates, organic compound, etc.. In one embodiment, the binding agent is an antibody ar a receptor which binds to an epitope of the biomarkers polypeptide.

Antibodies capable of binding molecules according the present invention preferable relate to antibodies which consist essentially of pooled monoclonal antibodies with different epitopic specificities, as well as distinct monoclonal antibody preparations. Monoclonal antibodies are made from synthetic or naturally occurring polypeptides according to the present invention by methods well known to those skilled in the art (Koehler et al. Nature 256(1975), 495). As used herein, the term "antibody" (Ab) or "monoclonal antibody" (mAb) is meant to include intact molecules as well as antibody fragments (such as, for example Fab and F(ab)₂ fragments) which are capable of specifically binding to polypeptides or derivatives thereof disclosed in the present invention. For certain purposes, e.g. diagnostic methods, the antibody or binding agent capable of binding of one or more of the inventive polypeptides or precursor proteins may be detectably labelled, for example with a radioisotope, a bioluminescent compound, a chemiluminescent compound, a fluorescent compound, a metal chelate, an enzyme or a mass tag specifically altering their mass. Furthermore, any method suitable for the detection of the intermolecular interaction may be employed.

The antibody or antigen binding agent is said to react specifically, if it reacts at a detectable level with a polypeptides disclosed herein, and does not significantly react with other polypeptides. The antibodies according to the present invention may be monoclonal or polyclonal antibodies. Other molecule capable of binding specifically may be for example antigen-binding fragments of antibodies such as Fab fragments, RNA, molecules and modifications thereof, DNA molecules and modifications thereof, RNA molecules and modifications thereof, or polypeptide and modifications thereof. According to the present invention binding agents may be used isolated or in combination. By means of combination it is possible to achieve a higher degree of sensitivity.

The antibody used according to the present invention may be coupled to one or more agents. The multiple agents coupled to one antibody may be all of the same species or may be several different agents bound to one antibody.

In one embodiment, the testing method incorporates an immunoassay, such as an ELISA type assay, that may employ any one of the above described antibodies or antibody fragments to detect in a sample material the presence (or up-regulation or down-regulation) of one or more biomarker polypeptides according to the present invention but in particular biomarker polypeptides exhibiting an amino acid sequence as shown in SEQ ID NOs: 1-42.

Other assays which may be used to detect in a sample material the presence (or up-regulation or down-regulation) of one or more biomarker polypeptides according to the present invention include a radioimmune assay (RIA), a Western blot assay, or a slot blot assay. The immunoassay can be used either as a simple detection method, or to measure the amount or level of biomarker polypeptides present. Such measurements can be quantitative, by comparing measured amounts to a reference standard representing a known or control amount of the marker, or can be used to compare relative amounts between different specimens or samples. The antibody may be free or immobilized on a substrate. The amount or presence of biomarker is typically determined via assay directed to a detectable biomarker or other indication of binding of the antibody to biomarker. Measures can be based on, for example, detection of fluorescence, luminescence, chemiluminescence, absorbance, reflectance, transmittance, birefringence or refractive index (e.g., surface plasmon resonance, ellipsometry, a resonant mirror method, a grating coupler waveguide method or interferometry). Optical methods include microscopy (both confocal and non-confocal), imaging methods and non-imaging methods. Electrochemical methods included voltametry and amperometry methods. Radio frequency methods include multipolar resonance spectroscopy. Methods for performing these assays are readily known in the art.

Other methods can be used to detect the presence or amount of a biomarker in a sample. Typically, the biomarker is first captured on a substrate. Examples of such methods include, but are not limited to, gas phase ion spectrometry methods, optical methods, electrochemical methods, atomic force microscopy and radio frequency methods. In one embodiment, the method comprises mass spectrometry, such as "surface-enhanced laser desorption/ionization" or "SELDI". SELDI refers to a method of desorption/ionization gas phase ion spectrometry (e.g., mass spectrometry) in which the analyse is captured on the surface of a SELDI probe that engages the probe interface. In "SELDI-MS," the gas phase ion spectrometer is a mass spectrometer. The invention also includes microarrays, such as oligonucleotide microarrays or cDNA microarrays, comprising the nucleic acid molecules encoding one or more of the 14 biomarkers provided in the invention. Such microarrays, can be employed in *in vitro* assays for assessing the expression level of the biomarker in the test cells from tumor biopsies, and determining the transformation stage of the test sample. For example, a microarray can be prepared using all the biomarkers, or subsets thereof, as described herein and shown in SEQ ID NOs: 1-42 and Table 1. Following application of nucleic acids isolated from both test samples and control samples, respectively, to one or more of the microarrays, the pattern of gene expression of the tested cells can be determined and compared with that of the biomarker pattern from the control panel.

In one embodiment, the invention provides diagnostic reagents that comprise one or more biomarkers probes, or one or more biomarker primers. A diagnostic reagent may comprise biomarker probes and/or biomarker primers from the same biomarkers gene or from multiple biomarkers genes. In another embodiment, the invention also provides diagnostic compositions that comprise one or more biomarker probes and target biomarker polynucleotides, or one or more biomarkers primers and target polynucleotides, or biomarkers primers, biomarker probes and biomarker target polynucleotides. In some embodiments, the diagnostic compositions comprise biomarkers probes and/or biomarkers primers and a samples suspected to comprise biomarker target polynucleotides. Such diagnostic compositions comprise biomarker probes and/or biomarkers primers and the nucleic acid molecules (including RNA, mRNA, cRNA, cDNA, and/or genomic DNA) of a subject in need of a diagnosis/prognosis of lung cancer.

In one embodiment of the invention, said diagnostic compositions are provided in form of diagnostic or therapeutic kits. Kits are, therefore, in certain embodiments also within the scope of the invention for use in the diagnostic and therapeutic applications described herein. The kits of the present invention can be used for clinical diagnosis and/or laboratory research. Such kits can comprise a carrier, package or container that is compartmentalized to receive one or more containers such as vials, tubes, and the like, each of the container(s) comprising one of the separate elements to be used in the method. For example, the container(s) can comprise a probe that is or can be detestably labelled. The probe can be an antibody or polynucleotide specific for a marker polypeptide or protein or a polynucleotide molecule comprising a nucleotide sequence encoding said marker polypeptide or protein. Alternatively, the kit can comprise a mass spectrometry (MS) probe. The kit can also include containers containing nucleotide(s) for amplification or silencing of a target nucleic acid sequence, and/or a container comprising a reporter-means, such as a biotin-binding protein, e.g., avidin or streptavidin, bound to a detectable label, e.g., an enzymatic, fluorescent, or radioisotope label. The kit can include all or part of the amino acid sequence of the marker polypeptide or protein, or a nucleic acid molecule that encodes such amino acid sequences.

Preferably, the kit also comprises instructions in any tangible medium on the use of the diagnostic reagent(s) in one or more methods of the invention.

The kit of the invention will typically comprise the container described above and one or more other containers comprising materials desirable from a commercial and user standpoint, including buffers, diluents, filters, needles, syringes, and package inserts with instructions for use. In one embodiment, the kit may comprise a suitable container that comprises one or more microarrays, e. g. , oligonucleotide microarrays or cDNA microarrays, which comprise those biomarkers for use in testing cells from patient tissue specimens or patient samples; and instructions for use.

In addition, kits contemplated by the invention can further include, for example, reagents or materials for monitoring the expression of biomarkers of the invention at the level of mRNA or protein, using other techniques and systems practiced in the art such as, for example, RT-PCR assays, which employ primers designed on the basis of one or more of the biomarkers described herein, immunoassays, such as enzyme linked immunosorbent assays (ELISAs), immunoblotting, e. g. , Western blots, or in situ hybridization, and the liko, as further described herein.

Such a kit may optionally provide in separate containers enzymes and/or buffers for reverse transcription, in vitro transcription, and/or DNA polymerization, nucleotides, and/or labeled nucleotides, including fluorochrome-labelled nucleotides. Also included in the kit may be positive and negative controls for the methods of the invention.

For protein-based methods, such as immunoassays, a diagnostic reagent in the kit may comprise a biomarkers antibody, which may be labeled, for example, by a fluorochrome. Such a kit may optionally provide in separate containers buffers, secondary antibodies, signal generating accessory molecules, labeled secondary antibodies, including fluorochrome-labeled secondary antibodies. The kit may also include unlabeled or labeled antibodies to various cell surface antigens which can used for identification or sorting of subpopulation of cells. Also included in the kit may be positive and negative controls for the methods of the invention. The positive and/or negative controls included in a kit can be nucleic acids, polypeptides, cell lysate, cell extract, whole cells from patients, or whole cells from cell lines.

In addition, a label can be provided on the container to indicate that the composition is used for a specific therapeutic or non-therapeutic application, and can also indicate directions for either *in vivo* or *in vitro* use, such as those described above. Directions and or other information can also be included on an insert which is included with the kit.

Such kits would be useful in a clinical setting for use in testing a patient's biopsied tumor or other cancer samples, for example, to determine or predict if the patient's tumor or cancer will be resistant or sensitive to a given treatment or therapy.

The foregoing description will be more fully understood with the reference to the following Examples. Such Examples are, however, exemplary methods of practicing the present invention and are not intended to limit the scope of the invention.

### EXAMPLES

### Example 1: Sample preparation using in vitro cultured cells

Three adeoncarcinoma cell lines: A549, NCI-H1703 (squamous cell adenocarcinoma) and NCI-H2085 are used as cells in the tumor group. NL20 (immortalized non-tumorigenic human bronchial epithelial cell line) and SAEC (small airway epithelial cells) are used as control cells . Cells have been grown according to state of the art techniques.

Cells are removed from tissue culture plates by use of trypsin/EDTA and a rubber policeman, centrifuged for 5 minutes at 600 g at room temperature and washed twice with PBS. For peptide extraction moist mass standardized samples of 50 000 to 400 000 cells are used. After addition of 200 µl of 200 mM acetic acid the samples are boiled for 10 minutes. Subsequently the pH of the samples is adjusted from pH 2-pH 3 using a 30 % stock solution of HCl and finally the samples are diluted to a final volume of 1.5 ml using a 0.1 % stock solution of TFA. After 10 minutes centrifugation at 18 000 g at 4 °C the supernatant is harvested and stored in tubes at -80°C until further processed. Cell extracts, corresponding to 50000 to 400 000 cells are used per chromatographic run.

### Example 2: Sample preparation using cell culture supernatants

Using a 10 ml syringe the cell culture supernatant is passed through a cellulose acetate filter with 0.2 µm pore size and 5 cm² filtration area (Minisart®, Sartorius AG, Goettingen, Germany). The pH of the filtered sample is adjusted to pH 2- pH 3 using a 30 % (vlv) stock solution of HCl and stored at -80 °C until further processed. 1.5 mL of cell culture supernatant is used per chromatographic run.

### Example 3: Liquid chromatography of the samples

The separation method carried out is reverse phase chromatography. Various RP chromatography resins and buffers are equally suitable. The separation of peptides and/or proteins using a reverse phase chromatography column having a size of size 4 mm x 250 mm is done as described below. Here, a Source RPC, 4,6 x 150 mm (Amersham Biosciences Europe GmbH, Freiburg, Germany) is used. Buffer A is 0.06 % volume per volume (v/v) Trirfluoroacatic sad (TFA) and 99.94 % (v/v) distilled water and buffer B is 0.05% (v/v) TFA, 80 % (v/v) acetonitrile and 19,95 % (v/v) distilled water, The chromatography took place at 37°C using a HP 1100 HPLC with a micro flow cell both supplied by Agilent Technologies, Boeblingen, Germany. The pH of the sample is adjusted to pH 2-pH 3 using a stock solution of 30% (v/w) MCI and subsequently the samples are dilute to 1.5 ml using a 0.05 % (v/v) stock solution of trifluoroacetic acid. Prior to chromatography the samples are centrifuged at 4° C at 18 000 g for 10 minutes and finally 1.5 ml of sample are loaded onto the chromatography column representing an equivalent of 50 000 to 400 000 in vitro cultured cells or 1.5 ml cell culture supernatant. Buffers A and B are mixed in various ratios during chromatography. The percentage of buffer A is 100% minus % buffer B (v/v). The chromatography conditions are as follows
- 5% buffer B for 1min
- a linear gradient from 5 to 50% buffer B during the next 44 min.
- a linear gradient from 50 to 100% buffer B during the next 4min
- 100% buffer B for the next 4min.
The flow rate is 500 ml/min. and 96 fractions, each containing 0.25 mL are collected during the gradients from 0 to 100% Buffer B

### Example 4: Mass spectrometry of the samples

For mass spectrometric analysis, typical positive ion spectra of peptides are measured using a MALDI-TOF (matrix-assisted laser desorption ionization time of flight) mass spectrometer. Suitable MALDI-TOF mass spectrometers among others are manufactured by PerSeptive Biosystems, Framingham, USA (Voyager-DE, Voyager-DE PRO or Voyager-DE STR) or by Bruker Daltonik, Bremen, Germany (BIFLEX). Typical matrix substances suitable for peptides are matrix substances which comprise an organic acid such as 3,5-dimethoxy-4-hydroxycinnamic acid, alpha-cyano-4-hydroxycinnamic acid and 2,5-dihydroxybenzoic acid. For MALDI sample preparation we used alpha-cyano-4-hydrox- cinnamic acid as matrix and 6-desoxy-I-galactose as co-matrix, dissolved in acetonitrile containing 0.1 % TFA. A lyophilized equivalent obtained by reverse phase chromatography corresponding to 1000 to 5000 of in vitro cultured cells or 30 µl cell culture supernatant spotted onto the mass spectrometric carrier plate is used to measure the peptides and/or proteins. The fractionated, lyophilized sample is dissolved in 15 µl of a matrix solution. This matrix solution contained, for example, 10 g/l α-cyano-4-hydroxycinnamic acid and 10 g/L L(-)fucose dissolved in a solvent mixture consisting of acetonitrile, water, trifluoroacetic acid (TFA) and acetone in a ratio of 49:49:1:1 by volume (v/v). 0.3 µl of this sample solution is transferred to a MALDI carrier plate, and the dried sample is analyzed in a Voyager-DE STR MALDI mass spectrometerfrom PerSeptive Biosystems. The measurement took place in linear mode with delayed extraction^{™}. A MALDI-TOF mass spectrometer can be employed to quantify peptides such as, for example, the peptides of the invention if these peptides are present in a concentration which is within the dynamic measurement range of the mass spectrometer, thus avoiding detector saturation. The ability of MALI mass spectrometry to quantitate individual peptide and/or protein mass signals is confirmed by adding known concentrations of 50 to 800 pmol peptides, such as neurotensin, into complex biological samples such as plasma samples. Subsequently these samples are separated by reversed phase chromatography and those fractions containing the added peptides are analyzed by mass spectrometry as described in the examples, There is a the linear correlation between the MALDI mass spectrometry mass signal intensity of for example the neurotensin peptide and the amount of the neurotensin peptide added to the plasma sample, indicating that MALDI mass spectrometry is suitable to quantitatively determine peptides in complex samples such as plasma.

### Example 5: Data analysis

Sixteen replicates of the five cell lines were harvested at a cell density of approximately 75% to 100%. Equivalents to the predetermined cell numbers were subjected to peptide analysis (A549 and NCI-HC2703: 800,000 cells; NL-20: 1,200,200 cells; NCI-H2085: 400,000 cells and SAEC: 500,000 cells). Subsequently to fractionation as described in example 2 or 3, each fraction is individually analyzed by MALDI mass spectrometry as described in example 8 resulting in 96 mass spectra for each sample. These 96 mass spectra are electronically combined to produce a peptide display. For each cell type 16 replicates have been prepared and peptide common to the tumor cells have been compared to both cell lines representing normal tissue. The x-axis of these peptide displays depicts the molecular mass, the y-axis- depicts the fraction number and the colour intensity (grey scale) represents the mass spectrometric signal intensity. The data of peptide displays may be pre-processed by adjusting for background noise and outliers may be removed from the analysis. Differences between peptide displays are calculated by subtracting peptide displays from each other electronically and/or by correlation analysis of mass signals intensities. For correlation analysis every mass signal intensity above a threshold mass signal intensity is tested for correlation to any other mass signal intensity above the same certain threshold of mass signal intensity. Detection of different (relative) concentrations of peptides and/or proteins is done by comparison of the mass spectrometric data (mass signal intensities) from samples to be compared. Peptide displays from cell extracts from in vitro cultured cells or from tissue culture supernatants from tumor or normal cells gave about 1000 to 2300 non-redundant peptide and/or protein mass signals.

A total of 31 control cell samples and 48 cancer cell samples are processed. With a threshold of the correlation coefficient of 0.6% less than 5% of all signals show a positive or negative correlation with the tumor cell type. A non-parametric Mann-Whitney U test is used to compare the two groups. 106 signals have a higher and 74 signals have a lower signal in the tumor group compared to the control group. The threshold is set high enough to select only robust peaks in the spectra. Various peptide and/or protein mass signals are identified significantly altered between tumor and normal cells. Table 1 summarizes the peptides identified and their sequences including posttranslational modifications thereof. Sequencing of selected peptides revealed that they relate to 14 different proteins.

**Table 1**

| **No.** | **Complete SwissProt_name (DE)** | (5 AA prior)**AA-Sequenz**(5 AA after) | **Modifications** |
|---|---|---|---|
| Figure 2 | Cathepsin D precursor (EC 3.4.23.5) position 387-411 | (LWILG)DVFIGRYYTVFDRDNNRVGFAEA ARL(-) LeuTrplleLeuGlyAspValPhelleGlyArgTyrTyr ThrValPheAspArgAspAsnAsnArgValGlyPhe AlaGluAlaAlaArgLeu (SEQ ID NO: 1) AspValPhelleGlyArgTyrTyrThrValPheAspArg AspAsnAsnArgValGlyPheAlaGluAlaAlaArg Leu (SEQ ID NO: 2) | |
| Figure 15 | Cathepsin D precursor (EC 3.4.23.5) position 170-190 | (SASAL)GGVKVERQVFGEATKQPGITF(IA AKF) SerAlaSerAlaLeuGlyGlyValLysValGluArg GlnValPheGlyGluAlaThrLysGlnProGlyIleThr PhelleAlaAlaLysPhe (SEQ ID NO: 25) GlyGlyValLysValGluArgGlnValPheGlyGlu AlaThrLysGlnProGlylleThrPhe (SEQ ID NO: 26) | |
| Figure 17 | Cathepsin D precursor (EC 3.4.23.5) position 170-200 | (SASAL)GGVKVERQVFGEATKQPGITFIA AKFDGILG(MAYPR) SerAlaSerAlaLeuGlyGlyValLysValGluArg GlnValPheGlyGluAlaThrLysGlnProGlyIleThr PhelleAlaAlaLysPheAspGlylleLeuGlyMetAla TyrProArg (SEQ ID NO: 29) GlyGlyValLysValGluArgGlnValPheGlyGlu AlaThrLysGlnProGlyIleThrPheIleAlaAlaLys PheAspGlyIleLeuGly (SEQ ID NO: 30) | |
| | Cathepsin D precursor (EC 3.4.23.5) position 321-334 | (APASA)LVRIPLHKFTSIRR(TMSEV) AlaProAlaSerAlaLeuValArgIleProLeuHisLys PheThrSerlleArgArgThrMetSerGluVal (SEQ ID NO: 39) LeuValArglleProLeuHisLysPheThrSerlleArg Arg (SEQ ID NO: 40) | |
| Figure 3 | Prothymosin alpha position 2-36 | (-M)SDAAVDTSSEITTKDLKEK KEVVEEA ENG RDAPAD(EENGE) MetSerAspAlaAlaValAspThrSerSerGlulleThr ThrLysAspLeuLysGluLysLysGluValValGlu GluAlaGluAsnGlyArgAspAlaProAlaAspGlu GluAsnGlyGlu (SEQ ID NO: 3) SerAspAlaAlaValAspThrSerSerGlulleThrThr LysAspLeuLysGluLysLysGluvalvalGluGlu AlaGluAsnGlyArgAspAlaProAlaAsp (SEQ ID NO: 4) | Acetyl (N-term) |
| Figure 4 | Cytochrome c oxidase subunit VIb isoform 1 position 1-85 | (-)AEDMETKIKNYKTAPFDSRFPNQNQTR NCWQNYLDFHRCQKAMTAKGGDISVCE WYQRVYQSLCPTSWVTDWDEQRAEGT FPGKI(-) AlaGluAspMetGluThrLyslleLysAsnTyrLys ThrAlaProPheAspSerArgPheProAsnGlnAsn GlnThrArgAsncysTrpGlnAsnTyrLeuAspPhe HisArgCysGlnLysAlaMetThrAlaLysGlyGly AsplleSerValCysGluTrpTyrGlnArgValTyrGln SerLeuCysProThrSerTrpValThrAspTrpAsp GluGlnArgAlaGluGlyThrPheProGlyLyslle (SEQ ID NO: 5) | Acetyl (N-term), 4 bridged Cys |
| Figure 6 | Cytochrome c oxidase subunit VIb isoform 1 position 1-85 | (-)AEDMETKIKNYKTAPFDSRFPNQNQT RNCWQNYLDFHRCQKAMTAKGGDISV GEWYQRVYQSLGPTSWVTDWDEQRAE GTFPGKI(-) AlaGluAspMetGluThrLyslleLysAsnTyrLys ThrAlaProPheAspSerArgPheProAsnGlnAsn GlnThrArgAsnCysTrpGlnAsnTyrLeuAspPhe HisArgCysGlnLysAlaMetThrAlaLysGlyGly AsplleSerValCysGluTrpTyrGlnArgValTyrGln SerLeuGysProThrSerTrpValThrAspTrpAsp GluGlnArgAlaGluGlyThrPheProGlyLyslle (SEQ ID NO: 8) | Acetyl (N-term), described, 4 bridged Cys |
| Figure 18 | Cytochrome c oxidase polypeptide Vb, mitochondrial [Precursor] position 32-129 | (AMRSM)ASGGGVPTDEEQATGLEREIML AAKKGLDPYNVLAPKGASGTREDPNLVP SISNKRIVGCICEEDNTSVVWFWLHKGEA QRCPRCGAHYKLVPQQLAH(-) AlaMetArgSerMetAlaSerGlyGlyGlyValPro ThrAspGluGluGlnAlaThrGlyLeuGluArgGlulle MetLeuAlaAlaLysLysGlyLeuAspProTyrAsn ValLeuAlaProLysGlyAlaSerGlyThrArgGlu AspProAsnLeuValProSerlleSerAsnLysArglle ValGlyCyslleCysGluGluAspAsnThrSerVal ValTrpPheTrpLeuHisLysGlyGluAlaGlnArg CysProArgCysGlyAlaHisTyrLysLeuValPro GlnGlnLeuAlaHis (SEQ ID NO: 31) AlaSerGlyGlyGlyValProThrAspGluGluGln AlaThrGlyLeuGluArgGlulleMetLeuAlaAlaLys LysGlyLeuAspProTyrAsnValLeuAlaProLys GlyAlaSerGlyThrArgGluAspProAsnLeuVal ProSerlleSerAsnLysArglleValGlyCyslleCys GluGluAspAsnThrSerValValTrpPheTrpLeu HisLysGlyGluAlaGlnArgCysProArgCysGly AlaHisTyrLysLeuValProGlnGlnLeuAlaHis (SEQ ID NO: 32) | |
| Figure 5 | Glyceraldehyde-3-phosphate dehydrogenase position 1-18 | (-)GKVKVGVNGFGRIGRLVT(RAAFN) GlyLysValLysValGlyValAsnGlyPheGlyArglle GlyArgLeuValThrArgAlaAlaPheAsn (SEQ ID NO: 6) GlyLysValLysValGlyValAsnGlyPheGlyArglle GlyArgLeuValThr (SEQ ID NO: 7) | |
| Figure 8 | Glyceradehyde-3-phosphate dehydrogenase position 310-334 | (HFVKL)ISWYDNEFGYSNRVVDLMAHM ASKE(-) HisPheValLysLeulleSerTrpTyrAspAsnGlu PheGlyTyrSerAsnArgValValAspLeuMetAla HisMetAlaSerLysGlu (SEQ ID NO: 11) lleSerTrpTyrAspAsnGluPheGlyTyrSerAsn ArgValValAspLeuMetAlaHisMetAlaSerLys Glu (SEQ ID NO 12) | |
| Figure 9 | Glyceraldehyde-3- | (VNGFG)RIGRLVTRAAFNSGKVDIV | |
| | phosphate dehydrogenase position 12-39 | AINDPFIDL(NYMVY) ValAsnGlyPheGlyArglleGlyArgLeuValThr ArgAlaAlaPheAsnSerGlyLysValAsplleValAla lleAsnAspProPhelleAspLeuAsnTyrMetVal Tyr (SEQ ID NO: 13) (SEQ ID NO: 14) | |
| Figure 11 | Glyceraldehyde-3-phosphate dehydrogenase position 310-326 | (HFVKL)ISWYDNEFGYSNRVVDL(MAHM A) HisPheValLysLeulleSerTrpTyrAspAsnGlu PheGlyTyrSerAsnArgValValAspLeuMetAla HisMetAla (SEQ ID NO: 17) lleSerTrpTyrAspAsnGluPheGlyTyrSerAsn ArgValValAspLeu (SEQ ID NO: 18) | |
| | Glyceraldehyde-3-phosphate dehydrogenase position 310-334 | (HFVKL)ISWYDNEFGYSNRVVDLMAHM ASKE(-) HisPheValLysLeulleSerTrpTyrAspAsnGlu PheGlyTyrSerAsnArgValValAspLeuMetAla HisMetAlaSerlysGlu (SEQ ID NO: 41) lleSerTrpTyrAspAsnGluPheGlyTyrSerAsn ArgValValAspLeuMetAlaHisMetAlaSerLys Glu (SEQ ID NO: 42) | Oxidation (M) |
| Figure 7 | Sequestosome-1 (Phosphotyrosine-independent ligand for the Lck SH2 domain of 62 kDa) position 13-25 | (AYLLG)KEDAAREIRRFSF(CCSPE) AlaTyrLeuLeuGlyLysGluAspAlaAlaArgGlulle ArgArgPheSerPheCysCysSerProGlu (SEQ ID NO: 9) | |
| Figure 10 | G2/mitotic-specific cyclin-B1 position 97-112 | (VPVSE)PVPEPEPEPEPEPVKE(EKLSP) ValProValSerGluProValProGluProGluPro GluProGluProGluProValLysGluGluLysLeu SerPro (SEQ ID NO: 15) ProValProGluProGluProGluProGluProGlu ProValLysGlu (SEQ ID NO: 16) | |
| Figure 12 | Parathymosin position 1-97 | (-)SEKSVEAAAELSAKDLKEK KEKVEEKA SRKERKKEVVEEEENGAEEEEEETAEDG EEEDEGEEEDEEEEEEDDEGPALKRAAE EEDEADPKRQKTEN(GASA-) SerGluLysSerValGluAlaAlaAlaGluLeuSer AlaLysAspLeuLysGluLysLysGluLysValGlu GluLysAlaSerArgLysGluArgLysLysGluVal ValGluGluGluGluAsnGlyAlaGluGluGluGlu GluGluThrAlaGluAspGlyGluGluGluAspGlu GlyGluGluGluAspGluGluGluGluGluGluAsp AspGluGlyProAlaLeuLysArgAlaAlaGluGlu GluAspGluAlaAspProLysArgGlnLysThrGlu AsnGlyAlaSerAla (SEQ ID NO: 19) SerGluLysSerValGluAlaAlaAlaGluLeuSer AlaLysAspLeuLysGluLysLysGluLysValGlu GluLysAlaSerArgLysGluArgLysLysGluVal ValGluGluGluGluAsnGlyAlaGluGluGluGlu GluGluThrAlaGluAspGlyGluGluGluAspGlu GlyGluGluGluAspGluGluGluGluGluGluAsp AspGluGlyProAlaLeuLysArgAlaAlaGluGluGluAspGluAlaAspProLysArgGlnLysThrGlu Asn (SEQ ID NO: 20) | Acetyl (N-term), described |
| Figure 13 | Nucleoside diphosphate kinase B (EC 2.7.4.6) (NDK B) position 2-22 | (M)ANLERTFIAIKPDGVQRGLVG(EIIKR) MetAlaAsnLeuGluArgThrPheIleAlaIleLysPro AspGlyValGlnArgGlyLeuValGlyGlullelleLys Arg (SEQ ID NO: 21) AlaAsnLeuGluArgThrPhelleAlalleLysProAsp GlyValGlnArgGlyLeuValGly (SEQ ID NO: 22) | Acetyl (N-term), described |
| Figure 14 | 40S ribosomal protein S11 position 2-26 | (M)ADIQTERAYQKQPTIFQNKKRVLLG (ETGKE) MetAlaAspIleGlnThrGluArgAlaTyrGlnLysGln ProThrllePheGlnAsnLysLysArgValLeuLeu GlyGluThrGlyLysGlu (SEQ ID NO: 23) AlaAsplleGlnThrGluArgAlaTyrGlnLysGlnPro ThrlePheGlnAsnLysLysArgValLeuLeuGly (SEQ ID NO: 24) | Acetyl (N-term) |
| Figure 16 | (P61024) Cyclin-dependent kinases regulatory subunit 1 (CKS-1) position 2-79 | (M)SHKQIYYSDKYDDEEFEYRHVMLPKD IAKLVPKTHLMSESEWRNLGVQQSQGWV HYMIHEPEPHILLFRRPLPKKPKK(-) MetSerHisLysGlnlleTyrTyrSerAspLysTyrAsp AspGluGluPheGluTyrArgHisValMetLeuPro LysAsplleAlaLysLeuValProLysThrHisLeu MetSerGluSerGluTrpArgAsnLeuGlyValGln GlnSerGlnGlyTrpValHisTyrMetlleHisGluPro GluProHislleleuLeuPheArgArgProLeuPro LysLysProLysLys (SEQ ID NO: 27) SerHisLysGlnlleTyrTyrSerAspLysTyrAsp AspGluGluPheGluTyrArgHisValMetLeuPro LysAsplleAlaLysLeuValProLysThrHisLeu MetSerGluSerGluTrpArgAsnLeuGlyValGln GlnSerGlnGlyTrpValHisTyrMetileHisGluPro GluProHislleLeuLeuPheArgArgProLeuPro LysLysProLysLys (SEQ ID NO: 28) | Acetyl (N-term) |
| Figure 19 | Keratin, type I cytoskeletal 18 position 415-429 | (RRIVD)GKVVSETNDTKVLRH(-) ArgArglleValAspGlyLysValValSerGluThrAsn AspThrLysValLeuArgHis (SEQ ID NO: 33) | |
| Figure 1 | 40S ribosomal protein S15 (RIG protein) position 1-26 | (-)AEVEQKKKRTFRKFTYRGVDLDQLLD (MSYEQ) AlaGJuValGluGlnLysLysLysArgThrPheArg LysPheThrTyrArgGlyValAspLeuAspGlnLeu LeuAspMetSerTyrGluGln (SEQ ID NO 35) AlaGluValGluGlnLysLysLysArgThrPheArg LysPheThrTyrArgGlyValAspLeuAspGlnLeu LeuAsp (SEQ ID NO: 36) | Acetyl (N-term) |
| | Beta-2-microglobulin precursor [Contains: Beta-2-microglobulin variant pl 5.3] position 21-119 | (SGLEA)IQRTPKIQVYSRHPAENGKSNFL NCYVSGFHPSDIEVDLLKNGERIEKVEHS DLSFSKDWSFYLLYYTEFTPTEKDEYACR VNHVTLSQPKIVKWDRDM(-) SerGlyLeuGluAlaIleGlnArgThrProLysIleGln ValTyrSerArgHisProAlaGluAsnGlyLysSer AsnPheLeuAsnCysTyrValSerGlyPheHisProSerAsplleGluValAspLeuLeuLysAsnGlyGlu ArglleGluLysValGluHisSerAspLeuSerPhe SerLysAspTrpSerPheTyrLeuLeuTyrTyrThr GluPheThrProThrGluLysAspGluTyrAlaCys ArgValAsnHisValThrLeuSerGlnProLyslleVal LysTrpAspArgAspMet (SEQ ID NO: 37) lleGlnArgThrProLyslleGlnValTyrSerArgHis ProAlaGluAsnGlyLysSerAsnPheLeuAsnCys TyrValSerGlyPheHisProSerAsplleGluVal AspLeuLeuLysAsnGlyGluArgIleGluLysVal GluHisSerAspLeuSerPheSerLysAspTrpSer PheTyrLeuLeuTyrTyrThrGluPheThrProThr GluLysAspGluTyrAlaCysArgValAsnHisVal ThrLeuSerGlnProLyslleValLysTrpAspArg AspMet (SEQ ID NO: 38) | complete protein; 2 bridged Cys |

## Claims

1. A method of detecting cancerous cells in a tissue sample, preferably a tissue sample isolated from the human or animal body by
a) determining in said sample the level of a biomarker polypeptide or a panel of biomarkers polypeptides which are differentially expressed in proliferating cells, particularly in proliferating cancerous cells as compared to pre-cancerous and non-cancerous, normal cells,
b) determining a reference value, and
c) comparing the level of the biomarker polypeptides or panel of biomarker peptides determined in step a) with the reference value determined in step b).

2. A method for diagnosing in an individual a disorder associated with abnormal cell proliferation comprising
a) obtaining from said individual to be tested an isolated sample material;
b) determining in said sample material the concentration of a biomarker polypeptides or a panel of biomarker polypeptides which are differential expressed in proliferating cells, particularly in proliferating cancerous cells as compared to pre-cancerous and non-cancerous, normal cells,
c) determining a reference value in a control sample known to contain pre-cancerous or non-cancerous cells the concentration of said same biomarker peptide or panel of biomarkers peptides as used in step b), and
d) comparing the level of the biomarkers polypeptides or panel of biomarker peptides determined in step b) with the reference value determined in step c).

3. A method according to any of the preceding claims, wherein the sample is obtained by bronchoaveolar lavage or derived from exhaled breath condensate.

4. A method according to any of the preceding claims, wherein the biomarker polypeptides comprises an amino acid sequence exhibiting at least 80%, particularly at least 85%, more particularly at least 90%, even more particularly at least 95%, but especially at least 98% and up to 99% sequence identity to an amino acid sequence selected from the group of sequences shown in SEQ ID NOs: 1-42.

5. A method according to any of the preceding claim, wherein the extent of the alteration between the value determined for the biomarker polypeptide and the reference value amounts to at least 200%, 300%, 400%, 500%, 600%, 700%, 800%, 900%, and 1000%.

6. A method according to any of the preceding claims, wherein proliferating cancerous cells are cells of a non-small lung cancer, particularly cells of a lung adenocarcinoma, a squamous cell carcinoma or a large cell carcinoma.

7. A method according to any of the preceding claims, wherein the level of the biomarker polypeptide or a panel of maker polypeptides which is associated with proliferating cancerous cells, particularly proliferating cancerous cells in non-small cell lung cancer, is detected using a test system for detecting said biomarker polypeptide.

8. The method of claim 7, wherein said test system for detecting the biomarkers polypeptide is selected from the group consisting of an antibody-based assay, a histological or cytological assay, an expression level assay and an aptamer-based assay.

9. The method of claim 8, wherein the biomarker polypeptide is detected by an expression level assay comprising determining expression of a biomarker gene by measuring the amount of biomarker messenger RNA by DNA-DNA hybridization, RNA-DNA hybridization, reverse transcription-polymerase chain reaction (PCR), or real time quantitative PCR.

10. The method of claim 9, wherein a polypeptide as depicted in SEQ ID NOs: 1-42, or a fragment thereof, is used as a hybridization probe or primer.

11. The method of claim 8, wherein the biomarker polypeptide is detected by an antibody-based assay, wherein an antibody or a binding fragment thereof is used which binds to an epitope of the biomarker polypeptide as depicted in SEQ ID NOs: 1-42.

12. A kit for use in any of the methods according to claims 1 to 11.

13. A panel of polypeptides comprising a plurality of polypeptide molecules each comprising an amino acid sequence selected from the group of sequences as shown in SEQ ID NOs: 1-42.

14. A panel of polypeptides according to claim 13, which comprises at least 2 polypeptide molecules each comprising an amino acid sequence selected from the group of sequences as shown in SEQ ID NOs: 1-42.

15. A panel of polypeptides according to claim 13, which comprises
a. at least 5 polypeptide molecules each comprising an amino acid sequence selected from the group of sequences as shown in SEQ ID NOs: 1-42; or
b. at least 10 polypeptide molecules each comprising an amino acid sequence selected from the group of sequences as shown in SEQ ID NOs: 1-42.
